# EUROPEAN PATENT APPLICATION

(11) **EP 1 801 117 A1**
(43) Date of publication of application: **27.06.2007**
(21) Application number: 05077955.2
(22) Date of filing: 21.12.2005
(51) Int. Cl.: C07K 14/315, C12N 1/21, C12N 15/31, C12P 1/04, C12P 13/06, C12P 13/12, A23C 9/12, A23L 1/03

(54) **Means and methods for regulating amino acid pools and/or transport**

(71) Applicant: Friesland Brands B.V., 7943 PE Meppel (NL)
(72) Inventor: Nauta, Arjen, 6721 CG Bennekom (NL); Kuipers, Oscar Paul, 9728 XG Groningen (NL); Kok, Jan, 9714 HL Groningen (NL); Sikkema, Jan, 9483 PC Zeegse (NL); den Hengst, Christiaan Daniël, 7418 ED Deventer (NL)
(74) Representative: Winckels, Johannes Hubertus F.

(57) **Abstract**

The invention provides a method for regulating the amount of at least one branched-chain amino acid in a cell, comprising regulating the amount and/or activity in said cell of a CtrA transporter and/or a homologue thereof, the amount and/or activity in said cell of a *Lactococcus lactis* BrnQ transporter and/or a homologue thereof, and/or the amount and/or activity in said cell of a functional part, derivative and/or analogue of at least one of said transporters and/or homologues. Said method preferably further comprises regulating the amount of at least one sulphur-comprising amino acid in a cell, comprising regulating the amount and/or activity in said cell of a CtrA transporter, a CtrA homologue, and/or a functional part, derivative and/or analogue of said CtrA transporter and/or CtrA homologue.

### Annex to the application documents - subsequently filed sequences listing

SEQUENCE LISTING

<110>  Friesland Brands B.V.

<120>  Means and methods for regulating amino acid pools and/or
       transport

<130>  P75404EP00

<140>  EP 05077955.2
<141>  2005-12-21

<160>  13

<170>  PatentIn version 3.3

<210>  1
<211>  30
<212>  DNA
<213>  Artificial Sequence

<220>
<223>  Primer

<400>  1
gctctagact agaattagga catatatcac                                        30


<210>  2
<211>  34
<212>  DNA
<213>  Artificial Sequence

<220>
<223>  Primer

<400>  2
cgcggatccc tcataaatcc cataataaat cctc                                   34


<210>  3
<211>  30
<212>  DNA
<213>  Artificial Sequence

<220>
<223>  Primer

<400>  3
cgcggatcct ggttccttat tggaattgcg                                        30


<210>  4
<211>  30
<212>  DNA
<213>  Artificial Sequence

<220>
<223>  Primer

<400>  4

ccggaattca acggctggta cgatacgaac 30

<210> 5
<211> 30
<212> DNA
<213> Artificial Sequence

<220>
<223> Primer

<400> 5
gctctagaca ttagtccaaa tggcgatacc 30

<210> 6
<211> 32
<212> DNA
<213> Artificial Sequence

<220>
<223> Primer

<400> 6
cgcggatccg atagtcttta ccagctagtt tc 32

<210> 7
<211> 32
<212> DNA
<213> Artificial Sequence

<220>
<223> Primer

<400> 7
cgcggatccg agcttctaat atttaggagc tc 32

<210> 8
<211> 32
<212> DNA
<213> Artificial Sequence

<220>
<223> Primer

<400> 8
ccggaattcc atctcggttt taacgtctga ac 32

<210> 9
<211> 31
<212> DNA
<213> Artificial Sequence

<220>
<223> Primer

<400> 9
ctagaccacc atgggattta tgagaaaagc c 31

<210>  10
<211>  32
<212>  DNA
<213>  Artificial Sequence

<220>
<223>  Primer

<400>  10
ctagtctaga cgtcttattt ctttttgcga cg                                    32


<210>  11
<211>  56
<212>  DNA
<213>  Artificial Sequence

<220>
<223>  Primer

<400>  11
ctagtctaga ttagtgatgg tgatggtgat gtttcttttt gcgacgattt ccataa          56


<210>  12
<211>  1680
<212>  DNA
<213>  Lactococcus lactis


<220>
<221>  CDS
<222>  (181)..(1578)

<400>  12
caccagaagt actgacagaa attgatttta ataaaataat ttactgacga atctgtcagt      60

atttttttga aaaaaatttt atactgcata aagttaattc ttgacaattg tctgacaatt     120

cggtaaaata cagtttattg aaattattta gttacaaatt cagaaaaatg aggatttatt     180

atg gga ttt atg aga aaa gcc gat ttt gag ctt tat cgt gat gca gat       228
Met Gly Phe Met Arg Lys Ala Asp Phe Glu Leu Tyr Arg Asp Ala Asp
1               5                   10                  15

aaa cat tat aat cag gta cta acc aca cgt gat ttt ctt gca cta ggt       276
Lys His Tyr Asn Gln Val Leu Thr Thr Arg Asp Phe Leu Ala Leu Gly
            20                  25                  30

gtt gga aca att att tca act tct atc ttt acc ttg cca gga caa gtt       324
Val Gly Thr Ile Ile Ser Thr Ser Ile Phe Thr Leu Pro Gly Gln Val
        35                  40                  45

gcc gct caa ttt gcg ggc cca ggg gtt gtc ttt tct tat cta ctt gcc       372
Ala Ala Gln Phe Ala Gly Pro Gly Val Val Phe Ser Tyr Leu Leu Ala
    50                  55                  60

gct ttg gtc gct ggt ttt gtg gcc ttg gct tat gct gaa atg tca aca       420
Ala Leu Val Ala Gly Phe Val Ala Leu Ala Tyr Ala Glu Met Ser Thr

65                  70                  75                  80

gta atg ccc ttt gct ggt tca gcc tat tct tgg att tcg gtc tta ttt      468
Val Met Pro Phe Ala Gly Ser Ala Tyr Ser Trp Ile Ser Val Leu Phe
                85                  90                  95

ggc gaa ggt ttc ggc tgg att gct ggt tgg gct ttg ctt gcg gaa tat      516
Gly Glu Gly Phe Gly Trp Ile Ala Gly Trp Ala Leu Leu Ala Glu Tyr
            100                 105                 110

ttt att gct gtt gcc ttt gtt ggc tca ggt ttc tca gca aat ctt cag      564
Phe Ile Ala Val Ala Phe Val Gly Ser Gly Phe Ser Ala Asn Leu Gln
        115                 120                 125

caa tta cta gcg cca ctc ggt ttc caa ctg cca aaa gta ttg gcc aac      612
Gln Leu Leu Ala Pro Leu Gly Phe Gln Leu Pro Lys Val Leu Ala Asn
    130                 135                 140

cct ttt ggt aca gat ggt gga atc gtt gat att att tca ctt ttg gtc      660
Pro Phe Gly Thr Asp Gly Gly Ile Val Asp Ile Ile Ser Leu Leu Val
145                 150                 155                 160

att ttg ctc tcg gca att att gtc ttt cgt ggg gca tct gat gct gga      708
Ile Leu Leu Ser Ala Ile Ile Val Phe Arg Gly Ala Ser Asp Ala Gly
                165                 170                 175

cga atc agt caa att ctt gtt gtg ctt aaa gtg gca gcg gtt att gct      756
Arg Ile Ser Gln Ile Leu Val Val Leu Lys Val Ala Ala Val Ile Ala
            180                 185                 190

ttc atc att gtg ggg att acg gtc att aag cca gca aat tac cat cca      804
Phe Ile Ile Val Gly Ile Thr Val Ile Lys Pro Ala Asn Tyr His Pro
        195                 200                 205

ttt att ccg cca cac aat ccg aaa aca ggt ttt ggt ggc ttc tcg ggg      852
Phe Ile Pro Pro His Asn Pro Lys Thr Gly Phe Gly Gly Phe Ser Gly
    210                 215                 220

att tgg tct ggg gtg tcg atg att ttc ttg gcc tat atc ggt ttt gat      900
Ile Trp Ser Gly Val Ser Met Ile Phe Leu Ala Tyr Ile Gly Phe Asp
225                 230                 235                 240

tca att gca gcc aat tca gcg gaa gct aaa aat ccc caa aaa aca atg      948
Ser Ile Ala Ala Asn Ser Ala Glu Ala Lys Asn Pro Gln Lys Thr Met
                245                 250                 255

cca cgt gga att att ggg tcg ctt ctc att gct gtt gtt cta ttt gca      996
Pro Arg Gly Ile Ile Gly Ser Leu Leu Ile Ala Val Val Leu Phe Ala
            260                 265                 270

gca gta acc ttg gta ctt gta ggg atg cat cct tac tca gcc tat gca      1044
Ala Val Thr Leu Val Leu Val Gly Met His Pro Tyr Ser Ala Tyr Ala
        275                 280                 285

gga aat gca gcg cca gtt ggt tgg gcc ctt caa caa tca ggt tat tct      1092
Gly Asn Ala Ala Pro Val Gly Trp Ala Leu Gln Gln Ser Gly Tyr Ser
    290                 295                 300

gtt tta tca gaa gtt gtc aca gcg att gca ctt gcc gga atg ttt atc      1140
Val Leu Ser Glu Val Val Thr Ala Ile Ala Leu Ala Gly Met Phe Ile
305                 310                 315                 320

gct ctt ttg gga atg gtc ctt gca ggt tcg cgt ttg ctt tat gct ttt      1188
Ala Leu Leu Gly Met Val Leu Ala Gly Ser Arg Leu Leu Tyr Ala Phe
                325                 330                 335

gga cgc gat gga ctt ttg cct aaa ggt tta ggt aaa atg aat gct cga      1236
Gly Arg Asp Gly Leu Leu Pro Lys Gly Leu Gly Lys Met Asn Ala Arg
            340                 345                 350

aat ctt cca gca aat ggt gtg tgg acc tta gcc att gtt gct att gtc      1284
Asn Leu Pro Ala Asn Gly Val Trp Thr Leu Ala Ile Val Ala Ile Val
        355                 360                 365

att ggg gca ttc ttc ccc ttt gct ttt ctt gct caa ctt atc tca gcc      1332
Ile Gly Ala Phe Phe Pro Phe Ala Phe Leu Ala Gln Leu Ile Ser Ala
    370                 375                 380

gga act ttg att gct ttc atg ttt gta act ctt gga att tat tcg ctc      1380
Gly Thr Leu Ile Ala Phe Met Phe Val Thr Leu Gly Ile Tyr Ser Leu
385                 390                 395                 400

cgt cgt cgt caa gga aaa gat tta cca gaa gca act tat aaa atg cct      1428
Arg Arg Arg Gln Gly Lys Asp Leu Pro Glu Ala Thr Tyr Lys Met Pro
                405                 410                 415

ttt tat cca gta cta cca gct ctt ggt ttt att ggg tca tta ttt gtc      1476
Phe Tyr Pro Val Leu Pro Ala Leu Gly Phe Ile Gly Ser Leu Phe Val
            420                 425                 430

ttt tgg gga ctg gat gtt caa gca aaa ctt tat tct gga att tgg ttc      1524
Phe Trp Gly Leu Asp Val Gln Ala Lys Leu Tyr Ser Gly Ile Trp Phe
        435                 440                 445

ctt att gga att gcg att tat ttt gct tat gga aat cgt cgc aaa aag      1572
Leu Ile Gly Ile Ala Ile Tyr Phe Ala Tyr Gly Asn Arg Arg Lys Lys
    450                 455                 460

aaa taa gacgaaaaat aaaaaaatta gcttagagat tacaaaaaat ggtttcaaaa      1628
Lys
465

ttcttgagac catttttttg tcattatgtt ggagctgaca aaaatggcta aa      1680


<210>  13
<211>  465
<212>  PRT
<213>  Lactococcus lactis

<400>  13

Met Gly Phe Met Arg Lys Ala Asp Phe Glu Leu Tyr Arg Asp Ala Asp
1               5                   10                  15


Lys His Tyr Asn Gln Val Leu Thr Thr Arg Asp Phe Leu Ala Leu Gly
            20                  25                  30


Val Gly Thr Ile Ile Ser Thr Ser Ile Phe Thr Leu Pro Gly Gln Val
        35                  40                  45

Ala Ala Gln Phe Ala Gly Pro Gly Val Val Phe Ser Tyr Leu Leu Ala
    50                  55                  60

Ala Leu Val Ala Gly Phe Val Ala Leu Ala Tyr Ala Glu Met Ser Thr
65                  70                  75                  80

Val Met Pro Phe Ala Gly Ser Ala Tyr Ser Trp Ile Ser Val Leu Phe
                85                  90                  95

Gly Glu Gly Phe Gly Trp Ile Ala Gly Trp Ala Leu Leu Ala Glu Tyr
            100                 105                 110

Phe Ile Ala Val Ala Phe Val Gly Ser Gly Phe Ser Ala Asn Leu Gln
        115                 120                 125

Gln Leu Leu Ala Pro Leu Gly Phe Gln Leu Pro Lys Val Leu Ala Asn
    130                 135                 140

Pro Phe Gly Thr Asp Gly Gly Ile Val Asp Ile Ile Ser Leu Leu Val
145                 150                 155                 160

Ile Leu Leu Ser Ala Ile Ile Val Phe Arg Gly Ala Ser Asp Ala Gly
                165                 170                 175

Arg Ile Ser Gln Ile Leu Val Val Leu Lys Val Ala Ala Val Ile Ala
            180                 185                 190

Phe Ile Ile Val Gly Ile Thr Val Ile Lys Pro Ala Asn Tyr His Pro
        195                 200                 205

Phe Ile Pro Pro His Asn Pro Lys Thr Gly Phe Gly Gly Phe Ser Gly
    210                 215                 220

Ile Trp Ser Gly Val Ser Met Ile Phe Leu Ala Tyr Ile Gly Phe Asp
225                 230                 235                 240

Ser Ile Ala Ala Asn Ser Ala Glu Ala Lys Asn Pro Gln Lys Thr Met
                245                 250                 255

Pro Arg Gly Ile Ile Gly Ser Leu Leu Ile Ala Val Val Leu Phe Ala
            260                 265                 270

Ala Val Thr Leu Val Leu Val Gly Met His Pro Tyr Ser Ala Tyr Ala
        275                 280                 285

Gly Asn Ala Ala Pro Val Gly Trp Ala Leu Gln Gln Ser Gly Tyr Ser
    290                 295                 300

Val Leu Ser Glu Val Val Thr Ala Ile Ala Leu Ala Gly Met Phe Ile
305                 310                 315                 320

Ala Leu Leu Gly Met Val Leu Ala Gly Ser Arg Leu Leu Tyr Ala Phe
                325                 330                 335

Gly Arg Asp Gly Leu Leu Pro Lys Gly Leu Gly Lys Met Asn Ala Arg
            340                 345                 350

Asn Leu Pro Ala Asn Gly Val Trp Thr Leu Ala Ile Val Ala Ile Val
        355                 360                 365

Ile Gly Ala Phe Phe Pro Phe Ala Phe Leu Ala Gln Leu Ile Ser Ala
    370                 375                 380

Gly Thr Leu Ile Ala Phe Met Phe Val Thr Leu Gly Ile Tyr Ser Leu
385                 390                 395                 400

Arg Arg Arg Gln Gly Lys Asp Leu Pro Glu Ala Thr Tyr Lys Met Pro
                405                 410                 415

Phe Tyr Pro Val Leu Pro Ala Leu Gly Phe Ile Gly Ser Leu Phe Val
            420                 425                 430

Phe Trp Gly Leu Asp Val Gln Ala Lys Leu Tyr Ser Gly Ile Trp Phe
        435                 440                 445

Leu Ile Gly Ile Ala Ile Tyr Phe Ala Tyr Gly Asn Arg Arg Lys Lys
    450                 455                 460

Lys
465

## Description

The invention relates to the field of biology and food production.

Various food products comprise microorganisms that provide desired characteristics to the product. For instance, dairy food production involves fermentation of a starter medium, mostly milk or a milk-derived product, by microorganisms which provide texture, taste and flavour characteristics to the final product. In dairy food production milk is often inoculated with lactic acid bacteria, which bacteria form part of many starter cultures used in yoghurt and cheese production. Lactic acid bacteria have a complex proteolytic system to break down the major milk protein casein into small peptides and amino acids that are necessary for growth in this medium. Initial breakdown of casein is carried out by the extracellular cell wall-bound serine proteinase PrtP.
Microorganisms comprise various transport systems which allow transfer of compounds between the microorganism and its environment. For instance, casein-derived peptides that are produced by the proteinase PrtP are internalised by lactic acid bacteria by peptide transporters, where after the peptides are further hydrolysed intracellularly into smaller peptides and amino acids by peptidases. Moreover, amino acid transport takes place via various amino acid transporters. Individual amino acids are subsequently converted into other compounds, such as for instance alcohols, aldehydes, acids, esters and sulphur compounds which involve specific flavour development.
Amino acid metabolism in microorganisms that are involved in food production contributes to the characteristics of the final food product. For instance, taste and flavour formation is often dependent on the metabolic pathways of micro-organisms. Hence, the characteristics of a food product are influenced by influencing amino acid metabolism of microorganisms present in said food product. Particularly, metabolism of branched chain amino acids and sulphur-comprising amino acids in microorganisms contribute to the characteristics of food products.

It is an object of the present invention to provide means and methods for influencing the amount of branched chain amino acids, and/or sulphur-comprising amino acids, in an organism. It is a further object to influence at least one characteristic of a food product.

In a first embodiment the invention provides a method for regulating the amount of at least one branched-chain amino acid and/or at least one sulphur-comprising amino acid in a cell, comprising regulating the amount and/or activity in said cell of a CtrA transporter, a CtrA homologue, a functional part, derivative and/or analogue of a CtrA transporter, and/or a functional part, derivative and/or analogue of a CtrA homologue.

A CtrA transporter has been described as a putative cationic amino acid transporter of *Lactococcus lactis.* The *ctrA* gene was designated as such because it contains domains that are conserved between the cationic amino acid permeases LysP, PotE and AnsP, which transport lysine, putrescine/ornithine and y-aminobutyrate, resectively (Bolotin et al, 2001; Marchler-Bauer et al, 2005)
According to the present invention, however, CtrA is capable of transporting branched-chain amino acids (such as isoleucine, leucine and valine) and sulphur-comprising amino acids (such as methionine and cysteine). This is surprising in view of the fact that CtrA contains domains that are conserved between the cationic amino acid permeases LysP, PotE and AnsP. In disagreement with its predicted function, and in spite of the homology between CtrA and cationic amino acid permeases, CtrA appears to have a different function. Preferably, the amount of isoleucine, leucine, valine and/or methionine is regulated because these amino acids are particularly well transported by CtrA or a homologue, functional part, derivative and/or analogue thereof. Most preferably the amount of isoleucine, leucine, and/or methionine is regulated.

The present invention furthermore provides the insight that *Lactococcus lactis* comprises a homologue of BrnQ, a low-affinity BCAA carrier in a number of Gram-positive bacteria, among which *Lactobacillus delbruckii* and *Salmonella typhimurium.* According to the present invention, this *L. lactis* BrnQ transporter is also capable of transporting branched chain amino acids across a cell membrane. Hence, the amount of a branched chain amino acid in a cell is in one embodiment regulated by regulating the presence, amount and/or activity of BrnQ, preferably *L.lactis* BrnQ, or a homologue, functional part, derivative and/or analogue thereof. Provided is therefore a method for regulating the amount of at least one branched-chain amino acid in a cell, comprising regulating the amount and/or activity in said cell of a BrnQ transporter, a BrnQ homologue, a functional part, derivative and/or analogue of a BrnQ transporter, and/or a functional part, derivative and/or analogue of a BrnQ homologue.

CtrA and BrnQ, as well as homologues, functional parts, derivatives and analogues thereof, are capable of transporting branched chain amino acids across a cell membrane. One embodiment of the invention thus provides a method for regulating the amount of at least one branched-chain amino acid in a cell, comprising regulating the amount and/or activity in said cell of a CtrA transporter and/or a homologue thereof, the amount and/or activity in said cell of a BrnQ transporter, preferably a *Lactococcus lactis* BrnQ transporter, and/or a homologue of BrnQ, and/or the amount and/or activity in said cell of a functional part, derivative and/or analogue of at least one of said transporters and/or homologues. Said method preferably further comprises regulating the amount of at least one sulphur-comprising amino acid in a cell, comprising regulating the amount and/or activity in said cell of a CtrA transporter, a CtrA homologue, and/or a functional part, derivative and/or analogue of said CtrA transporter and/or CtrA homologue

A branched chain amino acid is defined as an amino acid which comprises a branched side chain. Isoleucine, leucine and valine are three naturally occurring amino acids with a branched side chain. However, the term "branched chain amino acid" also encompasses other amino acid residues, for instance non-natural, unusual and/or modified amino acids comprising a branched side chain.
A sulphur-comprising amino acid is an amino acid that comprises at least one sulphur atom. A sulphur atom is present in the side chains of the common naturally occurring amino acids methionine and cysteine. However, the term "sulphur-comprising amino acid" also encompasses other amino acid residues, for instance non-natural, unusual and/or modified amino acids comprising a sulphur atom.

A method of the invention is suitable for regulating the amount of at least one BCAA and/or at least one sulphur-comprising amino acid in a cell. Preferably said cell comprises a cell of a micro-organism. More preferably said cell comprises a prokaryotic cell, more preferably a bacterial cell. In one embodiment said cell comprises (a cell of) a microorganism capable of fermenting milk and/or a milk-derived product, preferably Leuconostoc, Lactococcus, Lactobacillus, Streptococcus, Propionibacterium, Bifidobacterium, Pediococcus and/or Carnobacterium since these bacteria are particularly suitable for food production. In a particularly preferred embodiment said cell comprises a Lactococcus cell, most preferably a *Lactococcus lactis* cell.

A functional part of a CtrA transporter, of a BrnQ transporter, or of a homologue of CtrA and/or BrnQ is defined as a part which has at least one same property as said CtrA and/or BrnQ transporter or homologue in kind, not necessarily in amount. Said functional part is preferably capable of transporting branched chain amino acids across a cell membrane, albeit not necessarily to the same extent as CtrA and/or BrnQ. In one embodiment a functional part of a CtrA transporter, of a BrnQ transporter, or of a homologue of CtrA and/or BrnQ is part of a larger compound (preferably another compound than CtrA or BrnQ). For instance, a proteinaceous molecule (such as for instance a fusion protein) is provided which comprises a functional domain of a CtrA transporter, a functional domain of a BrnQ transporter, and/or a functional domain of a homologue of CtrA and/or BrnQ.
A functional derivative of a CtrA transporter, a BrnQ transporter, or a homologue of CtrA and/or BrnQ is defined as a transporter which has been altered such that at least one property - preferably a transport property - of the resulting compound is essentially the same in kind, not necessarily in amount. A derivative is provided in many ways, for instance through conservative amino acid substitution, whereby an amino acid residue is substituted by another residue with generally similar properties (size, hydrophobicity, etc), such that the overall functioning is likely not to be seriously affected.
A person skilled in the art is well able to generate analogous compounds of a CtrA transporter, a BrnQ transporter, or a homologue of CtrA and/or BrnQ. This is for instance done through screening of a peptide library. Such an analogue has essentially at least one same property as said CtrA transporter, BrnQ transporter, or homologue in kind, not necessarily in amount.
As used herein, the terms "CtrA", "BrnQ", "CtrA homologue" and "BrnQ homologue" also encompass a functional part, derivative and/or analogue of said CtrA, BrnQ and/or homologue. Moreover, the terms "CtrA" and "BrnQ" also encompass homologues thereof (unless explicitly stated otherwise).

CtrA comprises a *L. lactis* transporter. Various homologues of CtrA are known to be present in other microorganisms. However, their capability of transporting branched-chain amino acids and sulphur-comprising amino acids was not known until the present invention. A homologue of CtrA is defined herein as a (nucleic acid sequence encoding a) proteinaceous molecule which is a transporter capable of specifically transporting branched chain amino acids (and, preferably, also sulphur-comprising amino acids) across a cell membrane. A homologue of CtrA is specific for branched chain amino acids and, preferably, also for sulphur-comprising amino acids, meaning that other kinds of amino acids are significantly less efficiently transported across a cell membrane, if at all. For instance, *Bacillus subtilis* comprises the CtrA homologue YhdG. Table 3 depicts various homologues of *L.lactis* CtrA in several species of Gram-positive bacteria. One preferred embodiment therefore provides a method according to the invention wherein the amount and/or activity of a CtrA homologue as depicted in Table 3 is regulated.

Now that the invention provides the insight that CtrA, (*L.lactis*) BrnQ and homologues thereof are capable of transporting branched chain amino acids across a cell membrane, while CtrA and CtrA homologues are also capable of transporting sulphur-comprising amino acids across a cell membrane, the amount of at least one branched chain amino acid (BCAA) and/or at least one sulphur-comprising amino acid in a cell is regulated by regulating the presence, amount and/or activity of CtrA, BrnQ and/or a homologue thereof. CtrA, BrnQ and homologues thereof are capable of transporting branched chain amino acids into a cell as well as out of a cell, depending on the intracellular and extracellular concentrations. Moreover, CtrA and homologues thereof are capable of transporting sulphur-comprising amino acids into as well as out of a cell, depending on the intracellular and extracellular concentrations.

If an extracellular concentration of branched chain amino acids amino acids is high, CtrA, BrnQ and homologues thereof will transport branched chain amino acids into a cell. In such circumstances, if it is desired to lower the intracellular concentration of branched chain amino acids, the amount and/or activity of CtrA, BrnQ and/or a homologue thereof is preferably decreased. If it is however desired to increase the intracellular concentration of branched chain amino acids, the amount and/or activity of CtrA, BrnQ and/or a homologue thereof is preferably increased.
Moreover, if an extracellular concentration of sulphur-comprising amino acids is high, CtrA and/or a CtrA homologue will transport sulphur-comprising amino acids into a cell. In such circumstances, if it is desired to lower the intracellular concentration of sulphur-comprising amino acids, the amount and/or activity of CtrA and/or a CtrA homologue is preferably decreased. If it is however desired to increase the intracellular concentration of sulphur-comprising amino acids, the amount and/or activity of CtrA and/or a CtrA homologue is preferably increased.

If a cell is present in an environment wherein the extracellular concentration of branched chain amino acids is significantly lower than the intracellular concentration of branched chain amino acids, CtrA, BrnQ and homologues thereof will export branched chain amino acids out of the cell. In these circumstances, if it is desired to lower the intracellular concentration of branched chain amino acids, the amount and/or activity of CtrA, BrnQ and/or a homologue thereof is preferably increased, because in that case more branched chain amino acids will be transported out of the cell. As a consequence, if it is desired to increase the intracellular concentration of branched chain amino acids, the amount and/or activity of CtrA, BrnQ and/or a homologue thereof is preferably decreased so that transport of branched chain amino acids out of the cell is at least in part counteracted. Furthermore, if an extracellular concentration of sulphur-comprising amino acids is significantly lower than the intracellular concentration of sulphur-comprising amino acids, and if it is desired to lower the intracellular concentration of sulphur-comprising amino acids, the amount and/or activity of CtrA and/or a CtrA homologue is preferably increased because in that case more sulphur-comprising amino acids will be transported out of the cell. As a consequence, if it is desired to increase the intracellular concentration of sulphur-comprising amino acids, the amount and/or activity of CtrA and/or a CtrA homologue is preferably decreased so that transport of sulphur-comprising amino acids out of the cell is at least in part counteracted.
Hence, whether the amount and/or activity of CtrA, BrnQ and/or a homologue thereof needs to be increased or decreased depends on the intracellular and the extracellular concentration of branched chain amino acids and/or sulphur-comprising amino acids and on the desired amount of branched chain amino acids and/or sulphur-comprising amino acids within a cell.

Since a functional part, derivative and/or analogue of CtrA or of a CtrA homologue is also capable of transporting branched chain amino acids and sulphur-comprising amino acids across a cell membrane (albeit not necessarily to the same extent as CtrA or a CtrA homologue), the intracellular concentration of branched chain amino acids and/or sulphur-comprising amino acids is also regulated by regulating the amount of said functional part, derivative and/or analogue in a cell. In one embodiment a cell is provided with a functional part, derivative and/or analogue of a CtrA transporter and/or with a functional part, derivative and/or analogue of a homologue of CtrA. Depending on the extracellular and intracellular concentration of branched chain amino acids and/or sulphur-comprising amino acids, said functional part, derivative and/or analogue will either transport branched chain amino acids and/or sulphur-comprising amino acids into a cell, or excrete branched chain amino acids and/or sulphur-comprising amino acids out of a cell.

Furthermore, a functional part, derivative and/or analogue of BrnQ or of a BrnQ homologue is capable of transporting branched chain amino acids across a cell membrane (albeit not necessarily to the same extent as BrnQ or a BrnQ homologue). Hence, the intracellular concentration of branched chain amino acids is also regulated by regulating the amount of said functional part, derivative and/or analogue in a cell. In one embodiment a cell is provided with a functional part, derivative and/or analogue of a BrnQ transporter and/or with a functional part, derivative and/or analogue of a homologue of BrnQ. Depending on the extracellular and intracellular concentration of branched chain amino acids, said functional part, derivative and/or analogue will either transport branched chain amino acids into a cell, or excrete branched chain amino acids out of a cell.

In one preferred embodiment of the invention the presence of at least one branched chain amino acid in a cell is counteracted. The invention provides the surprising insight that a decline of at least one branched chain amino acid in a cell, preferably in a bacterial cell, more preferably in a micro-organism capable of fermenting milk or a milk-derived product, most preferably a *lactococcus* bacterium, results in an increased flavour formation by said cell. This is contrary to expectations, since branched chain amino acids are usually converted into flavour forming compounds. Hence, it would be expected that an amount of branched chain amino acids should be increased in order to obtain enhanced flavour formation. Nevertheless, according to the present invention a decline in the concentration of BCAA appears to improve flavour formation, as shown in the Examples. This effect is particularly apparent during dairy product formation. According to the present invention bacteria, preferably bacteria capable of fermenting milk or a milk-derived product, most preferably lactic acid bacteria, exhibit an enhanced flavour formation if their intracellular branched amino acid concentration is reduced. An enhanced flavour formation is preferred, amongst other thing because this way the time that is required for preparation and/or ripening of a dairy product such as yoghurt or cheese is decreased.

One embodiment provides a method according to the invention wherein the presence, amount and/or activity of a CtrA and/or BrnQ transporter and/or homologue thereof in a cell is counteracted. As explained above, if cells are grown in an environment with a high concentration of BCAA's as compared to the intracellular BCAA concentration, CtrA, BrnQ and/or a homologue thereof will transport BCAA's into the cell, resulting in an increase of the intracellular BCAA concentration. Hence, if an extracellular concentration of BCAA's is higher than the intracellular concentration of BCAA's, and if a low intracellular BCAA concentration is desired, for instance to enhance flavour production, the amount and/or activity of CtrA, BrnQ and/or a homologue thereof is preferably reduced, resulting in less uptake of branched chain amino acids into the cell. This embodiment is particularly suitable if bacteria, preferably bacteria capable of fermenting milk or a milk-derived product such as lactic acid bacteria, are grown in a medium with a relatively high concentration of branched chain amino acids such as for instance currently used synthetic media.

Various methods are known in the art for counteracting the presence, amount and/or activity of a CtrA and/or BrnQ transporter and/or homologue thereof. In one embodiment the amount of said transporter and/or homologue is decreased by functionally deleting at least part of a gene encoding said transporter and/or homolog. By functionally deleting at least part of a gene is meant that at least part of a gene is not, or to a significantly lesser extent, expressed. At least part of a gene is for instance functionally deleted by excising at least part of said gene from a (non-human) genome. This is for instance performed by homologous recombination. A CtrA and/or BrnQ knock out mutant of a microorganism, preferably a knock out mutant of a bacterium capable of fermenting milk or a milk-derived product such as lactococcus, is preferably generated, as exemplified in the examples for L.lactis MG1363. Such micro-organisms exhibit enhanced flavour formation. One aspect of the present invention therefore provides a bacterial cell, preferably a micro-organism capable of fermenting milk and/or a milk-derived product, more preferably a Lactococcus, Leuconostoc, Streptococcus, Lactobacillus, Propionibacterium, Bifidobacterium, Pediococcus and/or a Carnobacterium, more preferably a *Lactococcus lactis* cell, whose ctrA gene and/or brnQ gene is at least in part functionally deleted.
Said at least part of a gene is not necessarily excised as long as its capability of being translated is at least in part counteracted. For instance, small interfering RNA (siRNA) is used in order to degrade mRNA derived from said gene. siRNAs associate with various proteins to form the RNA-induced silencing complex (RISC), harbouring nuclease and helicase activity. The antisense strand of a siRNA guides the RISC to the complementary target RNA, and the nuclease component cleaves a target RNA in a sequence-specific manner. Hence, double stranded RNA is capable of inducing degradation of the homologous single stranded RNA in a host cell.
Alternatively, or additionally, expression of a gene encoding a CtrA and/or BrnQ transporter and/or homologue thereof is reduced by functionally deleting a promoter sequence or another activator of said gene. Many alternative methods are available in the art, which are well known and need no further discussion here. One embodiment therefore provides a method according to the invention wherein the amount of a CtrA transporter, BrnQ transporter and/or a homologue of CtrA or BrnQ is decreased by downregulating expression of a nucleic acid sequence encoding said transporter and/or homologue.

In a further embodiment the activity of a CtrA transporter, the activity of a BrnQ transporter and/or the activity of a homologue of CtrA or BrnQ is decreased by providing said cell with a compound capable of at least partially inhibiting a transportation activity of said transporter and/or homologue. Such compound for instance comprises an amino acid other than branched chain amino acids. If a transportation activity of a CtrA transporter and/or a CtrA homologue is to be reduced, said compound preferably comprises an amino acid other than BCAA and other than sulphur-comprising amino acids. Such other amino acids are capable of at least in part inhibiting a transportation activity of CtrA or BrnQ or a homologue thereof by binding said transporter. Such other amino acids are not transported, but block the binding site for BCAA's and, in case of CtrA or a CtrA homologue, the binding site for sulphur-comprising amino acids. Since said other amino acids have a lower affinity for CtrA, BrnQ or a homologue thereof as compared to BCAA's and/or sulphur-comprising amino acids, a relatively high (intracellular or extracellular) concentration of such other amino acids (as compared to the intracellular or extracellular concentration of BCAA's and sulphur-comprising amino acids) is preferred in order to reduce the activity of CtrA, BrnQ or a homologue thereof.

If cells are grown in an environment with a low concentration of BCAA's as compared to the intracellular concentration of BCAA's, the CtrA and/or BrnQ transporters, and/or homologues thereof, will transport BCAA's out of the cell, resulting in a decrease of the intracellular BCAA concentration. If a further decrease of BCAA is desired, said transportation of BCAA's out of the cell is preferably enhanced. Hence, if an extracellular concentration of BCAA's is lower than the intracellular concentration of BCAA's, and if a low intracellular BCAA concentration is desired, for instance to enhance flavour production, the amount and/or activity of CtrA, BrnQ and/or a homologue thereof is preferably enhanced, resulting in more secretion of branched chain amino acids and/or sulphur-comprising amino acids out of the cell into its environment. One embodiment therefore provides a method according to the invention wherein the presence, amount and/or activity in said cell of said transporter and/or homologue thereof is induced and/or enhanced. This embodiment is particularly suitable for microorganisms that are grown in milk or a milk-derived product, for instance during dairy product production.
As used herein, the term milk encompasses milk derived from any source. Preferably, milk is obtained from a mammal such as, but not limiting to, a cow, goat, sheep, mare and/or yak. In one embodiment of the invention milk comprises colostrum.
A milk-derived product is defined herein as a product resulting from any treatment of milk. A milk-derived product for instance comprises milk that has at least in part been fermented by a yoghurt producing culture, a buttermilk producing culture or a cheese-producing culture. In one embodiment a milk-derived product comprises cheese which is in its second ripening phase. Milk contains a low concentration of free amino acid. During milk fermentation, the major milk protein casein is broken down into peptides, which peptides are transported into milk-fermenting micro-organisms and intracellularly further cleaved into free amino acids. Hence, amino acid transport by CtrA and/or BrnQ is mainly directed out of the cell. Enhancing the transportation activity of CtrA, BrnQ and/or a homologue thereof thus results in increased secretion of branched chain amino acids out of the cells, resulting amongst other things in improved flavour formation.

Since CtrA and BrnQ are capable of transporting BCAA's across a cell membrane in both directions (i.e. into a cell as well as out of a cell), an equilibrium will tend to be established. This means that, if a micro-organism is grown in milk or a milk-derived product with a low BCAA concentration, CtrA and/or BrnQ (or a homologue thereof) will transport BCAA's out of the cells of said micro-organism, thereby raising the extracellular BCAA concentration and lowering the intracellular BCAA concentration. If the extracellular BCAA concentration increases too much, BCAA transportation out of the cells will diminish. In order to enhance (prolonged) BCAA secretion out of the cells, it is therefore preferred to reduce the availability of extracellular BCAA's. By reducing the availability of BCAA's is meant that free extracellular BCAA's are subjected to at least one kind of circumstances rendering them less available for uptake by said cells. For instance, said free BCAA's are bound to another compound to form a complex, which complex is less capable of being taken up by said cells.

In one embodiment the amount of CtrA, BrnQ and/or a homologue thereof, or the amount of a functional part, derivative and/or analogue thereof, is increased by providing said cell with a nucleic acid sequence encoding said transporter, homologue, functional part, derivative and/or analogue thereof. Many methods are known in the art for providing a cell with a nucleic acid sequence. For instance, calcium phosphate transfection, electroporation or liposome-mediated transfection is used. Alternatively, direct injection of the nucleic acid is employed or a nucleic acid is introduced into the cell by a vector, preferably a viral vector. A marker gene such as for instance antibiotic resistance is preferably used in identifying clones containing the introduced nucleic acid, as is well known in the art. Said nucleic acid sequence is preferably operably linked to a promoter which is active in said cell. In one preferred embodiment a Lactococcus cell is provided with a nucleic acid comprising a sequence encoding CtrA, BrnQ and/or a homologue, functional part, derivative and/or analogue thereof, which sequence is operably linked to a promoter that is active in Lactococcus. In one embodiment a constitutively active promoter is used (which remains active, see for instance Van der Vossen et al, 1987). Preferably however an inducible promoter is used (Kuipers et al, 1997). An inducible promoter is defined as a promoter whose activity is inducible by a human individual, preferably by regulating the amount of an exogenous compound. In one preferred embodiment a promoter that is inducible by nisin is used (see, for instance, De Ruyter et al, 1996). An increased amount of CtrA, BrnQ and/or a homologue, functional part, derivative and/or analogue thereof, results in increased transportation activity.

It is furthermore possible to increase the amount of (endogenous) CtrA, BrnQ and/or a homologue thereof by increasing transcription and/or translation of a gene encoding CtrA, BrnQ and/or a homologue thereof. For instance, en endogenous CtrA and/or BrnQ promoter is replaced by a stronger promoter. Preferably however the amount of a CtrA, and/or a homologue thereof is increased by at least in part inhibiting binding of CodY to a CodY binding domain capable of regulating expression of said transporter. CodY is a well-studied pleiotropic transcriptional regulator that was first identified in *Bacillus subtilis* as the nutritional repressor of the dipeptide-permease operon (Slack et al, 1995). Functional homologs of CodY are present in several Gram-positive bacteria in which the protein is involved in the regulation of a wide array of genes. A recent study has unraveled the genome-wide effects of CodY on gene expression of *Lactococcus lactis* (Den Hengst et al, 2005). In this lactic acid bacterium (LAB), the majority of the CodY-regulated genes is involved in the proteolytic system. In *B. subtilis* the activity of CodY is dependent on intracellular GTP levels, thereby sensing the energy state of the cell. In *L.lactis* this dependency on intracellular GTP levels has not been observed.
The *L.lactis ctrA* gene is one of the main targets of CodY. Transcription of *ctrA* is regulated by CodY. The upstream region of *ctrA* contains three copies of a conserved nucleotide stretch, termed CodY-box, that has recently been shown to serve as an operator site for CodY (Den Hengst et al, 2005). Electrophoretic mobility shift assays and DNaseI footprinting analyses revealed that CodY directly interacts with this sequence in the upstream region of *ctrA* (Den Hengst et al, 2005). *B. subtilis* contains a homolog of CtrA (i.e. YhdG). Like lactococcal *ctrA,* transcription of *yhdG* is strongly affected by CodY, as it was 116-fold derepressed in a *codY* deletion strain in a DNA microarray analysis (Molle et al, 2003).
CtrA expression is tightly regulated by CodY. If CodY binds to a CodY box upstream of ctrA, expression of ctrA is repressed. When binding of CodY to said CodY box is inhibited, ctrA expression is derepressed, resulting in an increased amount of CtrA. Hence, the amount of CtrA or a homologue thereof is increased by at least in part counteracting binding of CodY to a CodY binding domain capable of regulating expression of CtrA or a homologue thereof, resulting in derepression of CtrA expression. Binding of CodY to a CodY binding domain is for instance counteracted by at least in part deleting and/or modifying a CodY binding domain upstream of a ctrA gene and/or a gene encoding a CtrA homologue, which CodY binding domain is normally capable of regulating expression of a ctrA gene or a gene encoding a CtrA homologue.

Now that the invention provides the insight that CtrA, BrnQ and homologues, functional parts, derivatives and/or analogues thereof are capable of transporting branched chain amino acids across a cell membrane and that CtrA and CtrA homologues are capable of transporting sulphur-comprising amino acids across a cell membrane, it has become possible to regulate BCAA metabolism and/or sulphur-comprising amino acid metabolism in a cell. The invention therefore provides a method for regulating branched chain amino acid metabolism and/or sulphur metabolism of a cell comprising regulating the amount of at least one branched-chain amino acid and/or at least one sulphur-comprising amino acid in said cell with a method according to the invention.

As stated before, the invention provides the surprising insight that a decline of at least one branched chain amino acid in a cell results in an increased flavour formation by said cell. The invention thus provides a method for enhancing flavour formation by a cell comprising diminishing the amount of at least one branched chain amino acid in said cell as compared to a situation wherein flavour formation is not enhanced.
This effect is particularly apparent during dairy product formation. Milk-fermenting microorganisms, preferably lactic acid bacteria, surprisingly exhibit an enhanced flavour formation if their intracellular branched amino acid concentration is reduced. The invention therefore provides a method according to the invention, comprising the steps of enhancing the amount and/or activity of a CtrA transporter and/or a homologue thereof, and/or the amount and/or activity of a BrnQ transporter and/or a homologue thereof, in a micro-organism capable of fermenting milk or a milk-derived product, and inoculating milk or a milk-derived product with said micro-organism. Of course, said amount and/or activity does not need to be enhanced each time again. Once the activity of a CtrA, CtrA homologue, BrnQ and/or BrnQ homologue in a microorganism has been enhanced, said microorganism is preferably further cultured. A preferred culture is obtained with enhanced activity of a CtrA, CtrA homologue, BrnQ and/or BrnQ homologue. Said preferred culture is preferably used for inoculating milk or a milk-derived product.
Preferably a dairy product is produced. Dairy products produced with a method according to the invention have a stronger flavour within a given time span as compared to similar methods wherein wild type microorganisms are used. It has now become possible to produce a dairy product with a stronger flavour as compared to current dairy products. Alternatively, or additoinally, dairy products are now produced that need a shorter ripening time because flavour is formed more rapidly. The invention therefore provides a method for producing a dairy product comprising inoculating a starter medium with a culture capable of converting a starter medium into a dairy product, wherein the amount and/or activity of a CtrA transporter and/or a homologue thereof, and/or the amount and/or activity of a BrnQ transporter and/or a homologue thereof, in said culture is increased, and fermenting said starter medium. Said starter medium preferably comprises milk (for instance colostrum) or a milk-derived product.
Preferably a bacterial cell is used in a method according to the invention, more preferably a micro-organism capable of fermenting milk and/or a milk-derived product, most preferably a Lactococcus, Leuconostoc, Streptococcus, Lactobacillus, Propionibacterium, a Bifidobacterium, a Pediococcus and/or a Carnobacterium. Most preferably *L.lactis* is used.
Preferably, the concentration of branched amino acids is kept and/or brought below 8 mM, more preferably 4 mM, in *Lactococcus lactis* and/or below 2 mM, more preferably below 1.5 mM, in Bacillus subtilis.

As explained herein before, since CtrA and BrnQ are capable of transporting BCAA's across a cell membrane in both directions, an equilibrium will tend to be established. If milk or a milk-derived product is inoculated with a culture having an enhanced activity of CtrA, BrnQ and/or a homologue thereof, BCAA's will be transported out of the cell and accumulate in said milk or milk-like product until the extracellular BCAA concentration approximates the intracellular BCAA concentration. If the extracellular BCAA concentration increases too much, BCAA secretion out of the cells will diminish. In order to enhance (prolonged) BCAA secretion out of the cells, it is therefore preferred to reduce the availability of extracellular BCAA's.
The availability of extracellular BCAA's is reduced in various ways. In one embodiment said milk or milk-derived product is inoculated with a second culture which transports extracellular BCAA's into their cells. Hence, the extracellular BCAA concentration remains low since extracellular BCAA's are removed from said milk or milk-derived product by said second culture. Alternatively, or additionally, milk or a milk-derived product is provided with a compound capable of binding free BCAA's to form a complex, which complex is less capable of being taken up by said a culture having an enhanced activity of CtrA, BrnQ and/or a homologue thereof. Of course, said second culture and said compound are preferably chosen such that the resulting dairy product is suitable for (preferably human) consumption. Hence, said second culture and said compound are preferably not harmful.
Many alternative methods for reducing the availability of extracellular BCAA's are available in the art. These methods are suitable as long as the resulting dairy product remains suitable for (preferably human) consumption.

Milk-fermenting micro-organisms such as for instance *L.lactis* are capable of breaking down casein into peptides which are transported into the cell and intracellularly broken down into amino acids. Since casein-derived peptides comprise BCAA's, breakdown of these peptides will enhance the intracellular BCAA concentration, resulting in a decrease in flavour formation. In order to counteract this increase in intracellular BCAA concentration, one embodiment provides a method according to the invention wherein milk or a milk-derived product is pretreated in order to deprive said milk or milk-derived product at least in part of BCAA. This is for instance performed by breaking down casein, for instance by administering at least one enzyme capable of cleaving casein, followed by removal of at least one peptide comprising BCAA's from said milk or milk-derived product. Said peptide is for instance removed by filtration. Preferably, at least one peptide with a higher relative amount of BCAA's as compared to other peptides is removed. In this embodiment milk or a milk-derived product is obtained that has a lower BCAA content as compared to untreated milk or milk-derived product. Subsequently, said obtained milk or milk-derived product with a low BCAA content is preferably inoculated with a culture having an enhanced activity of CtrA, BrnQ and/or a homologue thereof. This way, flavour formation is enhanced since said culture having an enhanced activity of CtrA, BrnQ and/or a homologue thereof is capable of secreting more BCAA's into the milk or milk-derived product with low BCAA content before an equilibrium is reached.

In one embodiment, milk or a milk-derived product is firstly at least in part fermented by at least one culture capable of breaking down casein. Breakdown of casein results in a milk-derived product with a lower BCAA content as compared to milk. Subsequently, said milk-derived product with a lower BCAA content is inoculated with a second culture having an enhanced activity of CtrA, BrnQ and/or a homologue thereof. Said second culture will transport BCAA's out of the cells. Since the BCAA content of said milk-derived product is lower than the BCAA content of fresh milk, said second culture having an enhanced activity of CtrA, BrnQ and/or a homologue thereof is capable of secreting more BCAA's into said milk-derived product before an equilibrium is reached. As a result, flavour formation is enhanced.

In one embodiment a micro-organism having an enhanced activity of CtrA, BrnQ and/or a homologue thereof is firstly cultured in a medium other than milk, preferably an artificially made medium, which medium has a low BCAA content. As a result, flavour formation by said culture is enhanced. Subsequently, said culture is administered to milk or to a milk-derived product. Said milk-derived product is for instance obtained by fermenting milk by another kind of culture. After administration of said culture having an enhanced activity of CtrA, BrnQ and/or a homologue thereof, a final product is obtained with enhanced flavour.
In one embodiment said culture having an enhanced activity of CtrA, BrnQ and/or a homologue thereof is used to further ferment said milk or milk-derived product.

Embodiments of the present invention are optionally combined in order to provide a method specifically adapted to individual circumstances and (economic) needs.

In both *L. lactis* and *B. subtilis,* BCAAs modulate the activity of CodY by increasing the affinity of CodY for its binding sites. Binding of CodY to a CodY binding site represses expression of a gene controlled by said binding site. An increase in BCAA results in increased binding of CodY to a CodY binding site and repression of CodY controlled genes. A decrease of BCAA's results in derepression of said CodY controlled genes. In view of this dependency of CodY on BCAA's, the invention provides a method for regulating expression of a CodY controlled nucleic acid sequence in a cell by regulating the amount and/or activity of a BCAA transporter according to the invention. Provided is therefore a method for regulating expression of a CodY controlled nucleic acid sequence in a cell, comprising regulating the amount and/or activity in said cell of a CtrA transporter and/or a homologue thereof, the amount and/or activity in said cell of a *L.lactis* BrnQ transporter and/or a homologue thereof, and/or the amount and/or activity in said cell of a functional part, derivative and/or analogue of at least one of said transporters and/or homologues.
In Table 4, various genes are depicted whose expression is regulated by the amount and/or activity of a CtrA transporter, a BrnQ transporter and/or a homologue, functional part, derivative and/or analogue thereof. The first 20 lines of the table depicts genes whose expression is upregulated when the amount of a CtrA transporter, a BrnQ transporter and/or a homologue, functional part, derivative and/or analogue thereof is decreased. The other genes are genes whose expression is downregulated when the amount of a CtrA transporter, a BrnQ transporter and/or a homologue, functional part, derivative and/or analogue thereof is decreased. Further provided is therefore a method for regulating expression in a cell of at least one gene depicted in Table 4, comprising regulating the amount and/or activity in said cell of a CtrA transporter and/or a homologue thereof, the amount and/or activity in said cell of a brnQ transporter and/or a homologue thereof, and/or the amount and/or activity in said cell of a functional part, derivative and/or analogue of at least one of said transporters and/or homologues. Preferably, expression of a dppA, dppP, dppB, dppC, dppD, dppF, ybbE, llmg_0328, comX, gltA, citB, icD, leuC, leuA, yecA, chiA, yucG, rcfB, yxbD, argH and/or yjjA gene is regulated with a method according to the invention since these genes are particularly sensitive for regulation by CtrA, BrnQ and/or a homologue thereof.

As is shown in Table 4, expression of genes that are controlled by CmbR is regulated by the amount and/or activity of a CtrA transporter, a BrnQ transporter and/or a homologue, functional part, derivative and/or analogue thereof. Provided is therefore a method for regulating expression of a CmbR controlled nucleic acid sequence in a cell, comprising regulating the amount and/or activity in said cell of a CtrA transporter and/or a homologue thereof, the amount and/or activity in said cell of a *L.lactis* BrnQ transporter and/or a homologue thereof, and/or the amount and/or activity in said cell of a functional part, derivative and/or analogue of at least one of said transporters and/or homologues.

Table 4 comprises, amongst other things, genes that are not preceded by a CodY binding site. These genes are also depicted in Table 5. Even though the genes depicted in Table 5 are not preceded by a CodY binding site, their expression is nevertheless regulated by the intracellular amount of BCAA's and/or sulphur-comprising amino acids. The invention therefore further provides a method for regulating expression in a cell of at least one gene as depicted in Table 5, comprising regulating the amount of at least one branched-chain amino acid and/or sulphur-comprising amino acids in said cell.

One of the transcriptional units whose expression is regulated by the amount and/or activity of CtrA and/or a CtrA homologue is the dipeptide transport system dppA, P, B, C, D and F. This is shown in Table 4. When the intracellular BCAA and/or sulphur-comprising concentration is low, the expression of the dipeptide transport system dppA, P, B, C, D and F is enhanced (Table 4). Hence, peptide transport is influenced by the amount of BCAA and/or sulphur-comprising amino acids.
One embodiment of the invention therefore provides a method for regulating the amount of peptides in a cell, and/or peptide uptake by said cell, comprising regulating the amount of at least one branched chain amino acid in said cell. Further provided is a method for regulating the amount of peptides in a cell, and/or peptide uptake by said cell, comprising regulating the amount of at least one sulphur-comprising amino acid in said cell. The invention furthermore provides a method for regulating the amount of peptides in a cell, and/or peptide uptake by said cell, comprising regulating the amount and/or activity in said cell of a CtrA transporter and/or a CtrA homologue.

A low intracellular BCAA concentration results in an increase of peptide uptake. If these peptides comprise BCAA's, which is the case if milk is fermented, the intracellular BCAA concentration will raise again. In such case peptide uptake therefore counteracts a low intracellular BCAA concentration. If a low intracellular BCAA concentration is desired, for instance for enhancing flavour formation, it is preferred to use a micro-organism having an enhanced activity of CtrA, BrnQ and/or a homologue thereof and having an at least partly impaired dipeptide transport system dppA, P, B, C, D and F. If such micro-organism is used for fermenting milk or a milk-derived product, BCAA's are transported out of the cell while uptake of BCAA-containing peptides is at least in part prevented. This way an intracellular BCAA concentration will remain low and flavour formation will be enhanced.

Further provided is an isolated or recombinant cell comprising a diminished amount and/or activity of a CtrA transporter and/or a homologue thereof, and/or an altered amount and/or activity of a brnQ transporter and/or a homologue thereof, as compared to a natural situation. Said cell is preferably devoid of CtrA. A cell comprising a diminished amount and/or activity of a CtrA transporter and/or a homologue thereof is particularly suitable for maintaining a low intracellular BCAA and sulphur-comprising amino acid concentration when said cell is present in an environment with a high BCAA and sulphur-comprising amino acid concentration, as explained before, because transportation of BCAA and sulphur-comprising amino acids into the cell is at least in part inhibited. A cell comprising an altered amount and/or activity of a BrnQ transporter and/or a homologue thereof is suitable for maintaining and/or inducing either a high or a low intracellular concentration of BCAA and sulphur-comprising amino acids, depending on the extracellular BCAA and sulphur-comprising amino acid concentration, as explained herein before.
One embodiment provides an isolated or recombinant cell according to the invention, wherein expression of a CtrA transporter and/or a homologue thereof, and/or expression of a BrnQ transporter and/or a homologue thereof, is downregulated. Expression of CtrA, BrnQ and/or a homologue thereof is preferably downregulated by functionally deleting at least part of a gene coding therefore. Further provided is therefore an isolated or recombinant cell according to the invention wherein at least part of a nucleic acid sequence encoding a CtrA transporter and/or a homologue thereof, and/or a BrnQ transporter and/or homologue thereof, is functionally deleted.

If microorganisms such as milk-fermenting bacteria are grown in milk, the extracellular BCAA and sulphur-comprising amino acid concentration is low. In that case, CtrA, BrnQ and homologues thereof transport BCAA and/or sulphur-comprising amino acids across the cell membrane out of the cell. In order to lower the intracellular BCAA concentration of such cell, the amount and/or activity of CtrA, BrnQ and/or a homologue thereof is preferably upregulated. The amount of these transporters is preferably upregulated by providing a cell with a nucleic acid sequence coding therefore. Further provided is therefore an isolated or recombinant cell comprising an exogenous nucleic acid sequence encoding a CtrA transporter and/or a homologue thereof, and/or encoding a BrnQ transporter and/or a homologue thereof, and/or encoding a functional part, derivative and/or analogue of at least one of said transporters and/or homologs. An isolated or recombinant cell wherein expression of BrnQ and/or a homologue thereof is upregulated is also herewith provided, as well as an isolated or recombinant cell obtainable by a method according to the present invention.

Yet another aspect of the invention provides a dairy product comprising a micro-organism wherein the amount of at least one branched chain amino acid is decreased as compared to a natural micro-organism. As explained hereinbefore, flavour formation in said dairy product is enhanced. A dairy product according to the present invention preferably comprises a micro-organism wherein the amount and/or activity of a CtrA transporter and/or a homologue thereof, and/or the amount and/or activity of a BrnQ transporter and/or a homologue thereof, in said micro-organism is increased as compared to a natural micro-organism. Said micro-organism preferably comprises a culture capable of converting a starter medium into a dairy product, preferably a Leuconostoc, Lactococcus, Lactobacillus, Streptococcus, Propionibacterium, Bifidobacterium, Pediococcus and/or Carnobacterium species.
The amount and/or activity of CtrA, BrnQ and/or a homologue thereof is increased in various ways, as already explained herein.

Further provided is a use of a compound capable of regulating the amount and/or activity of a CtrA transporter and/or a homologue thereof, and/or the amount and/or activity of a BrnQ transporter and/or a homologue thereof, for regulating the amount of at least one branched-chain amino acid and/or sulphur-comprising amino acids in a cell. Such compound for instance comprises a compound capable of binding CtrA, BrnQ, a homologue of CtrA or BrnQ, or CodY.

The invention furthermore provides an expression system comprising a cell comprising:
- CodY or a homologue, functional part, derivative and/or analogue thereof, and
- CtrA, BrnQ or a homologue, functional part, derivative and/or analogue of CtrA and/or BrnQ, and
- a heterologous nucleic acid sequence of interest operably linked to a CodY binding domain. Expression of said heterologous nucleic acid of interest is regulated by regulating the amount and/or activity in said cell of said CtrA transporter, BrnQ transporter and/or said homologue, functional part, derivative and/or analogue of CtrA and/or BrnQ. The invention therefore provides a method for regulating expression of a nucleic acid sequence of interest in a cell, comprising regulating the amount and/or activity in said cell of a CtrA transporter, a BrnQ transporter and/or a homologue, functional part, derivative and/or analogue of CtrA and/or BrnQ.

The invention is further explained in the following example. This example does not limit the scope of the invention, but merely serves to clarify the invention.

### Example 1

### Materials and methods

**Bacterial strains, plasmids and growth conditions.** Strains and plasmids used in this study are listed in Table 1. *Lactococcus lactis* was grown in 2-fold diluted M17 broth (Terzaghi et al, 1975) supplemented with 0.5% glucose (½GM17) at 30°C or on ½GM17 solidified with 1.5% agar. When appropriate, 5 µgml⁻¹ of erythromycin and/or chloramphenicol (both from Sigma Chemical Co., St. Louis, MO) were added. Alternatively, cells were grown in a chemically defined medium (CDM), prepared as described earlier (Mierau et al, 1994), supplemented with 1% casitone (Difco Laboratories, Detroit, MI) or with specific dipeptides where indicated. Growth in 96-wells plates incubated at 30°C was monitored using a GENios microtiter plate reader (Tecan, Grödig, Austria). The A₅₉₅ of the cultures was measured every 30 min following 15 s of shaking.

**DNA manipulation, molecular cloning and transformation.** Routine DNA manipulations were performed as described (Sambrook et al, 1989). Total chromosomal DNA from *L. lactis* was extracted as described previously (Leenhouts et al, 1990). Minipreparations of plasmid DNA from *L. Lactis* were made using the High Pure Plasmid isolation Kit from Roche Molecular Biochemicals (Mannheim, Germany). Restriction enzymes and T4 DNA ligase were purchased from Roche Molecular Biochemicals. PCR amplifications were carried out using *Pwo* DNA polymerase (Roche Molecular Biochemicals) and oligonucleotides listed in Table 2. PCR products were purified with the High Pure PCR product purification kit (Roche Molecular Biochemicals). Electrotransformation of *L. lactis* was performed using a Bio-Rad Gene Pulser (Bio-Rad Laboratories, Richmond, CA) as described earlier (Holo et al, 1995).

**Construction of *L. lactis* deletion mutant strains.** DNA fragments containing approximately 600-bps of the flanking regions of the *bcaP* gene of *L. lactis* MG1363 were obtained by PCR using oligonucleotide pairs ctrA-P1/ctrA-P2 and ctrA-P3/ctrA-P4 (Table 2), cut with the appropriate restriction endonucleases and ligated into plasmid pORI280, a conditionally replicating vector (Leenhouts et al, 1996). The resulting plasmid, pORIΔ*bcaP*, was introduced in *L. lactis* MG1363 by electroporation together with the helper plasmid pVE6007. Following chromosomal integration of pORIΔ*bcaP*, cells were grown for about 60 generations under non-selective conditions after which a clone, designated as MG*bcaP*, was obtained in which *bcaP* was deleted. The *bcaP* mutation was confirmed by PCR and Southern blot analysis (Sambrook et al, 1989) using the ECL direct nucleic acid labeling system (Amersham Pharmacia Biotech, Little Chalfont, UK). *L. lactis* MG1363 strains in which either *brnQ* or both *brnQ* and *bcaP* are deleted (MG*brnQ* and MG*bcaPbrnQ*, respectively), were obtained using the same double cross-over strategy, with primer combinations brnQ-Pl/brnQ-P2 and brnQ-P3/brnQ-P4 for the deletion of *brnQ* (Table 2).

**Overproduction and immunodetection of native and histidine-tagged BcaP.** Full-length *bcaP* of *L. lactis* MG1363 was amplified from the chromosome by PCR using oligonucleotides ctrA-N and ctrA-C (Table 2), introducing 5' *Nco*I and 3' *Xba*I restriction enzyme sites, respectively. Alternatively, a sequence encoding a hexa-histidine-tag was fused to the 3'-end of *bcaP* using oligonucleotides ctrA-N and ctrA-CH6. The purified PCR products were digested with NcoI and XbaI and ligated downstream of the nisin-inducible promoter P*nisA* using the corresponding sites in pNG8048, resulting in pNG*bcaP* and pNG*bcaP-H6*, respectively. The plasmids were introduced into *L. lactis* strains NZ9000, MG1363 or MG*bcaP*, together with plasmid pNZ9530 (Kleerebezem et al, 1997) in the latter two strains, to enable nisin-induced production of BcaP or BcaP-H6, as described previously (De Ruyter et al, 1996).

Production of BcaP-H6 was detected by Western hybridization. To this end, samples were taken from cultures growing in ½GM17 containing the appropriate antibiotics. After 2 hrs of induction with 1000-fold diluted supernatant of an overnight culture of the nisin producing *L. lactis* strain NZ9700 (Kuipers et al, 1993), approximately 10⁸ cells were harvested by centrifugation, resuspended in 100 µl Birnboim solution A (Sambrook et al, 1989) containing 1 mg/mL lysozyme (Merck KGaA, Darmstadt, Germany) and incubated for 15 min at 48°C. Proteins were separated on 10% PAA gels by SDS-PAGE as described previously (Laemmli, 1970) and transferred to PVDF membranes (Roche Molecular Biochemicals). BcaP-H6 was visualized with specific anti-HIS antibodies and peroxidase-anti-mouse conjugates (both from Amersham Biosciences) according to the supplier's instructions.

**Amino acid transport assays.** *L. lactis* MG1363 (pNG*bcaP*; pNZ9530), *L. lactis* MG1363 or *L. lactis* MG*bcaP*, the latter two harboring plasmids pNG8048 and pNZ9530, were grown at 30°C in 50 ml of ½GM17. At an OD₆₀₀ of approximately 0.4, 1000⁻¹ volume of supernatant of the nisin-producing strain *L. lactis* NZ9700 (Kuipers et al, 1993) was added to the cultures to induce BcaP production. At an OD₆₀₀ of approximately 1.0, cells were harvested by centrifugation at 6300 g and 4°C. Cell pellets were washed with 25 ml of ice-cold CDM, concentrated to an OD₆₀₀ of 10.0 in CDM lacking amino acids and stored on ice until use. Cells were energized prior to transport assays by 5 min of incubation at 30°C in the presence of 0.5% glucose. Subsequently, 100 µl of cell suspension were mixed with 100 µL of CDM containing 40 µM of the unlabeled amino acid of interest and a final concentration of 1 µM of the same amino acid in a ¹⁴C- or ³⁵S-labeled form (Amersham Biosciences). For competition experiments, a 10-fold excess of unlabeled amino acid was added. Mixtures were incubated at 30°C for various time intervals while stirring. The reactions were stopped by the addition of 2 ml of ice-cold 0.1 M LiCl, followed by filtration through 0.45 µm pore-size nitrocellulose filters (Schleicher & Schuell, GmbH, Dassel, Germany). Reaction tubes and filters were washed with another 2 mL of ice-cold 0.1 M LiCl. Subsequently, the filters were transferred to vials containing 2 ml of scintillation fluid (Packard BioScience, Groningen, the Netherlands) and radioactivity was determined using a Packard TriCarb 2000 CA liquid scintillation analyzer (Packard Instruments, Meriden, CT).

**DNA microarray analysis.** DNA microarray experiments were performed essentially as described previously (Den Hengst et al, 2005). Briefly, RNA was isolated from four separately grown cultures of *L. lactis* MG1363 and *L. lactis* MG*bcaP.* Subsequently, single-strand reverse transcription (amplification) and indirect labeling of total RNA, with either Cy3- or Cy5-dye (Amersham Biosciences), were performed in duplicate. Labeled cDNA samples were hybridized to slides containing amplicons representing 2450 ORFs of *L. lactis* MG1363, spotted in duplicate. After hybridization, slides were washed and scanned. Slide data were processed and normalized as described previously (van Hijum et al, 2005), yielding average ratios of gene expression of the mutant over the wild type strain. Expression of a gene was considered to be significantly altered when its ratio of expression in the mutant compared to the wild type was >1.5 or <0.67 and had a *p*-value <0.001.

### Results

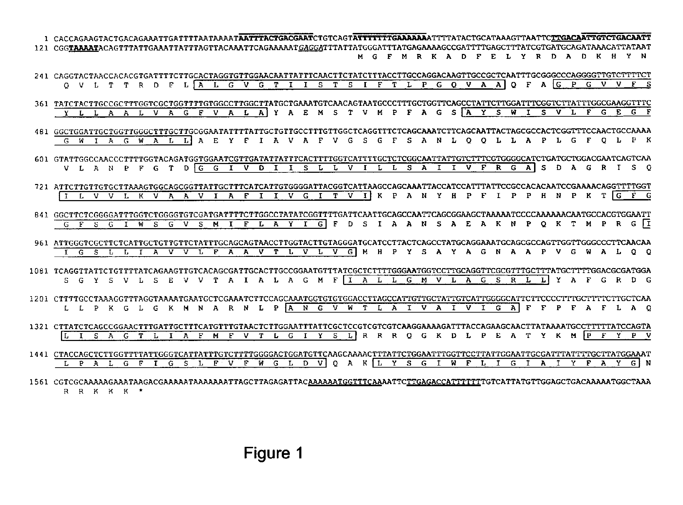

**Identification of the *L. lactis* MG1363 *beaP* (*ctrA*) locus**. A gene encoding the putative amino acid transporter CtrA was previously identified in a genome-wide screening for CodY targets in *L. lactis* MG1363 (Den Hengst et al, 2005). The results presented below show that CtrA is in fact a branched-chain amino acid permease. Therefore, we propose to rename the gene and protein accordingly into *bcaP* and BcaP, respectively. The 1,398 nucleotides-containing *bcaP* ORF encodes a protein of 465 amino acid residues with a predicted molecular mass of 49,6 kDa and an isoelectric point of 9.91 (Fig. 1). The *bcaP* ORF starts at an AUG codon following a proper lactococcal ribosome-binding site (GAGGA). Regions that could serve as promoter elements are present upstream of the start codon. These putative -35 (TTGACA) and -10 (TAAAAT) sequences are separated by 17-base pairs (bps) and are likely to constitute the promoter, as DNA fragments comprising this region can readily drive transcription (Den Hengst et al, 2005). In addition, three copies of a CodY-responsive element (CodY-box) can be discerned in the regulatory region of *bcaP.* Downstream of *bcaP,* a palindromic sequence could form a stem-loop structure with a predicted free energy value of -13.8 kcal/mol that could serve as a rho-independent transcriptional terminator.

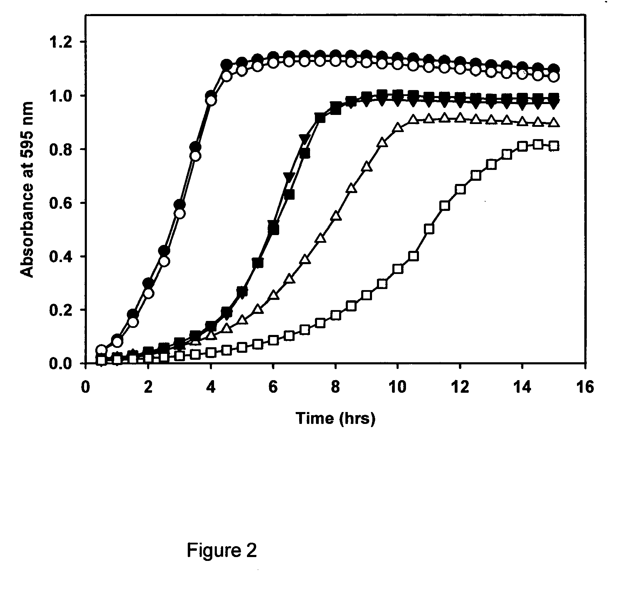

***bcaP* is required for optimal growth *of L. lactis* in media containing free amino acids as the sole amino acid source**. As transcription of *bcaP* is tightly regulated by the global regulator CodY in both *L. lactis* and *B. subtilis* and the protein is conserved among several Gram-positive bacteria, we wondered which biological function it serves. To address this question, the entire *bcaP* gene was removed from the chromosome of *L. lactis* MG1363 by double cross-over recombination. The mutated strain, MG*bcaP*, was examined for its ability to grow in media with different amino acid or peptide contents (Fig. 2). Growth of strains MG1363 and MG*bcaP* was similar in CDM containing casitone. This nitrogen source consists of a tryptic digest of milk caseins and contains a wide variety of peptides of different lengths and composition as well as free amino acids. The absence of a growth difference between the strains in this medium indicates that *bcaP* does not serve any apparent physiological function during growth under nitrogen-rich conditions. When growth was monitored in CDM containing a mixture of all 20 amino acids, but lacking peptides as amino acid source, strain MG*bcaP* behaved differently: it reached a lower final cell density and the maximum growth rate was significantly reduced compared to the wild type strain (Fig. 2). When the amino acid concentration was lowered to 75% of that in CDM (CDM⁷⁵), growth of strain MG*bcaP* was even more hampered (Fig. 2), while the wild type strain showed normal growth rates and final cell densities (data not shown). These results show that the presence of *bcaP* is required for optimal growth *of L. lactis* in media containing limiting amounts of amino acids.

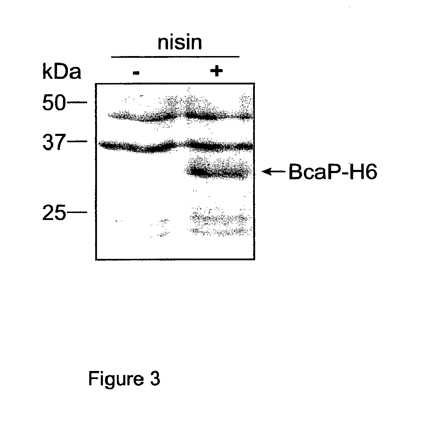

**Phenotypic complementation of the *bcaP* gene deletion.** In order to rule out the possibility that the observed differences in growth were due to polar effects on transcription of neighboring genes that could have been introduced through construction of the mutant, *L. lactis* MGbcaP was complemented by full-length *bcaP*. To this end, the *bcaP* gene was placed under control of a nisin-inducible promoter in plasmid pNG8048 and introduced into the *bcaP* mutant strain. However, no protein band corresponding to BcaP could be detected by SDS-PAGE after induction of BcaP synthesis with nisin. Therefore, a histidine tag was fused to the 3'-terminus of *bcaP* and proper BcaP synthesis was verified by SDS-PAGE followed by Western hybridization (Fig. 3). A protein band specific for histidine-tagged BcaP (BcaP-H6) could indeed be detected by Western analysis upon induction of BcaP-H6 synthesis. The size of the protein in this band corresponded to a lower molecular mass than the predicted 50.4 kDa for BcaP-H6, but it has been reported before that membrane proteins can migrate relatively fast in SDS-PAGE (Steffes et al, 1992). As can be seen in Fig. 2, normal growth was restored when native BcaP synthesis was induced in MG*bcaP* (pNG*bcaP*) grown in CDM containing the free amino acid mixture. These results show that *bcaP* can be provided in *trans* to complement MG*bcaP* and that polar effects of the *bcaP* mutation on growth are absent.

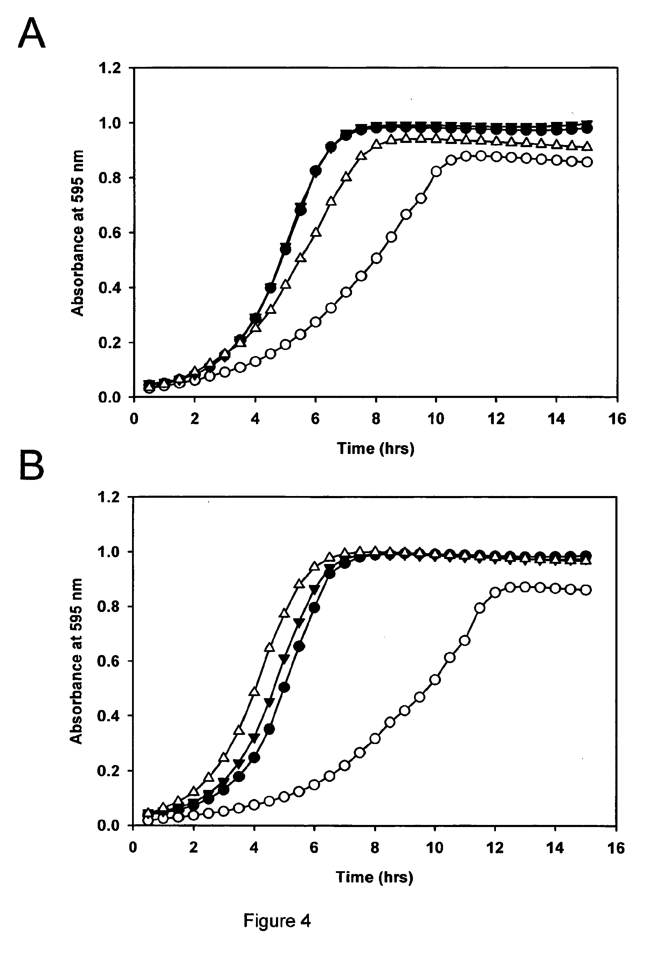

**A *bcaP* mutation is bypassed by addition of the BCAAs or BCAA-containing dipeptides to the growth medium.** The experiments described above indicated that *bcaP* encodes a functional protein that is required for optimal growth in the presence of free amino acids. To find out whether BcaP facilitates amino acid transport, as assumed from BLAST results and, if so, to gain insight in its substrate specificity, growth experiments with *MGbcaP* were carried out in media with different amino acid concentrations. Reasoning that the absence of BcaP in the mutant strain would likely result in suboptimal intracellular levels of its substrate(s), addition of an excess of these is expected to restore normal growth, provided an alternative internalization pathway (e.g. another carrier) is present. Based on homology, BcaP is predicted to transport cationic amino acids, hence its original name CtrA (Bolotin et al, 2001). However, no stimulatory effect on growth of MG*bcaP* was observed when the medium was supplemented with lysine, arginine, putrescine, ornithine or γ-aminobutyrate at a concentration of 5 mM of each of these compounds (data not shown). When 5 mM of the three BCAAs (leucine, isoleucine and valine) were added together, both the maximum cell density and growth rate of *MGbcaP* were increased significantly (Fig. 4A). Addition of 15 mM BCAAs fully restored growth (data not shown). In contrast, growth was not enhanced when the medium was supplemented with proline, alanine and histidine (Fig. 4A) and all 14 other non-branched chain amino acids (data not shown).

In accordance with these data, addition of a mixture of BCAA-containing dipeptides (i.e. LI and PV) fully restored growth of MG*bcaP,* whereas such a stimulatory effect was absent when dipeptides lacking BCAA residues were provided (Fig. 4B). The growth defect of MG*bcaP* was already bypassed at a concentration of 500 µM of the dipeptides, most probably because *L. lactis* has multiple highly efficient transport systems for dipeptides and contains several intracellular peptidases that generate free amino acids from dipeptides (Kok et al, 2003).

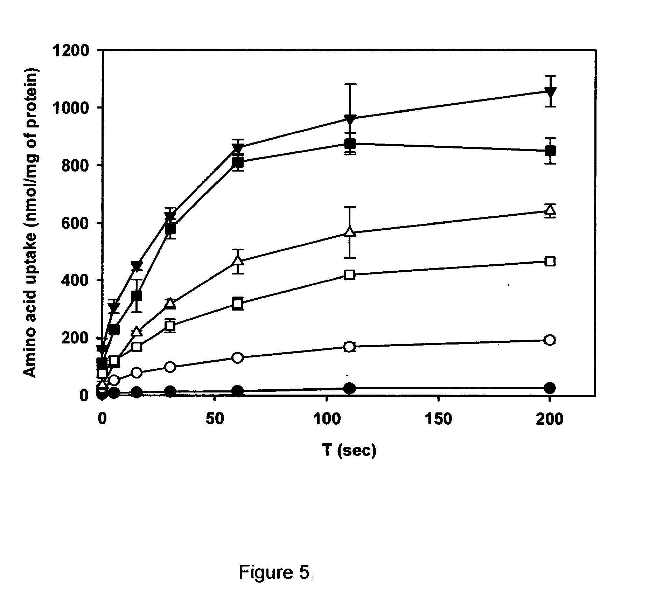

**BcaP specifically transports branched-chain amino acids and, to a lesser extent, methionine**. Since growth of the *bcaP* mutant could be restored by increasing BCAA concentrations in the medium, the lactococcal *bcaP* gene could encode a BCAA transporter. To test this possibility, the ability of the *L. lactis* MG1363 and MG*bcaP* to transport radioactively labeled L-isoleucine was compared (Fig. 5). Deletion of *bcaP* resulted in a reduction of the initial rate of isoleucine transport of approximately 9-fold, indicating that BcaP is responsible for most of the isoleucine uptake in *L. lactis* under these conditions. Complementation of BcaP function by overproducing BcaP in MG*bcaP* resulted in a strong increase (app. 70-fold) in the uptake rate of L-[¹⁴C]isoleucine. Increasing the ratio of L-[¹⁴C]isoleucine over unlabeled isoleucine, by lowering the concentration of unlabeled isoleucine in the reaction mixture from 250 to 50 µM, resulted in a more rapid internalization of L-[¹⁴C]isoleucine followed by a slower efflux of the isotope (data not shown). Such kinetics is indicative of so-called counterflow transport and suggests that BcaP is able to facilitate bidirectional transport, thus constituting a secondary transporter.

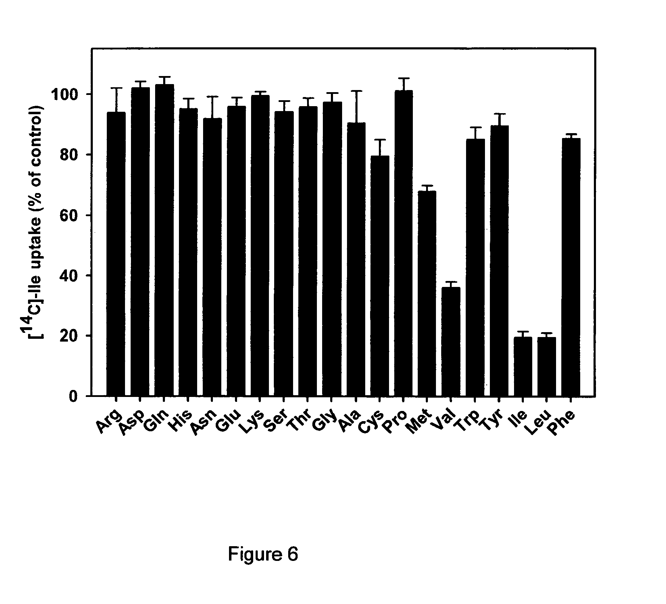

To estimate the substrate specificity of BcaP, inhibition of isoleucine transport by other amino acids was monitored in a competition experiment (Fig. 6). Addition of a 10-fold excess of isoleucine or leucine resulted in a reduction of uptake of about 80%, whereas valine was less inhibitory (64%). In addition to the BCAAs, transport of isoleucine by BcaP was inhibited by the sulfur-containing amino acids methionine (23% inhibition) and, to a lesser extent, cysteine. These results indicate that the ability of BcaP to bind amino acids increases as a function of their hydrophobicity, with the exception of those containing bulky side-chains (i.e. Trp, Tyr and Phe).

To test whether BcaP is capable of transporting the amino acids it recognizes, time course experiments were performed as described for isoleucine, using radioactively labeled leucine, valine and methionine. Consistent with the competition experiments, the transport rate of BcaP for leucine was comparable to that for isoleucine and the rate for valine was higher than that for methionine (Fig. 5). Uptake rates for these substrates by the *bcaP* mutant strain were similar to that of isoleucine (data not shown). No significant difference in transport of the non-binding amino acid proline was observed between the *bcaP* mutant and the BcaP-overproducing strain.

**BcaP is conserved in several Gram-positive bacteria**. The transport and growth experiments clearly show that BcaP specifically transports branched-chain amino acids and methionine, which is in disagreement with its predicted function. A BLAST search (Altschul et al, 1990) with the full-length amino acid sequence against the NCBI non-redundant protein database revealed that homologs of BcaP are contained in the genomes of a several Gram-positive bacteria (Table 3). The amino acid sequence of lactococcal BcaP is similar to that of YhdG of *B. subtilis.* Like *bcaP,* transcription of *yhdG* has been shown to be strongly CodY-dependent (Molle et al, 2003). This indicates that *L. lactis* BcaP and *B. subtilis* YhdG represent functional homologs. Homologs of BcaP are present in species of *Listeria*, *Streptococcus, Staphylococcus* and *Lactobacillus,* which all contain (putative) homologs of CodY (Sonenshein, 2005). Notably, in the latter bacterium a second homologue was found that is even more similar to *Lactococcal* BcaP, albeit that it has a C-terminal extension of about 34 amino acid residues that is not present in the *L. lactis* protein.

The amino acid sequences of the BcaP homologs are extensively conserved throughout the proteins, with the exception of the N-terminal parts. A hydropathy analysis of BcaP, using the method of Kyte and Doolittle (Kyte et al, 1982), predicts 12 transmembrane segments (boxed in Fig. 1). These transmembrane helices, with an average size of 23 amino acid residues, are linked by short hydrophilic stretches varying in size from 3 to 40 residues. Both the N- and C-terminus are predicted to protrude into the cytoplasm. The hydrophobicity profile of BcaP is conserved in all homologs. Among the 465 amino acid residues constituting BcaP, 326 are hydrophobic (70%), 87 are polar (18.7%), 18 are acidic (3.9%), and 34 are basic (7.3%) amino acid residues, respectively. Thus, BcaP is strongly hydrophobic, which is a typical feature of integral membrane transporters.

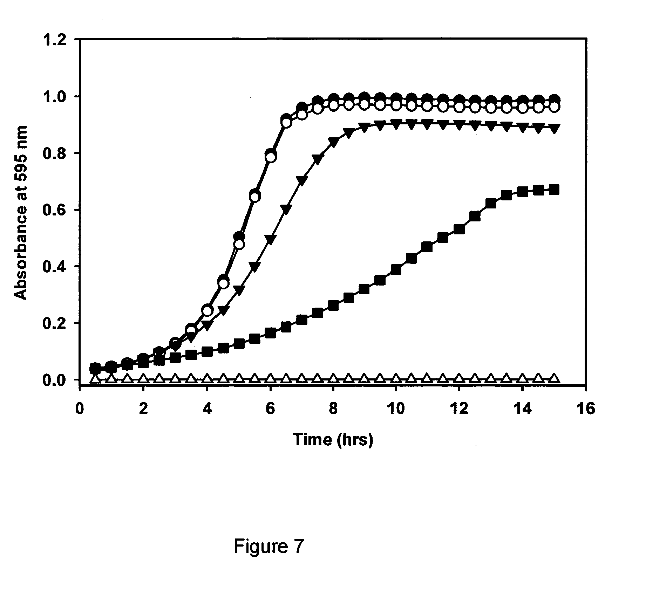

**The role of BcaP in global gene expression in *L. lactis.*** As transcription of *bcaP* is tightly regulated by the global regulator CodY and the absence of *bcaP* is deleterious for the cells under amino acid limiting conditions (Fig. 2), the effect of removal of *bcaP* on global gene expression was monitored by comparing the transcriptomes of *L. lactis* MG1363 and *L. lactis* MG*bcaP*. Both strains were grown in CDM containing free amino acids as sole amino acid source. RNA samples were prepared from each strain and, following cDNA synthesis and labeling, hybridized to DNA microarrays representing 2450 genes of *L. lactis* MG1363. Analysis of the DNA microarray data of four biological replicates revealed that the expression of approximately 40 genes was significantly influenced by the *bcaP* mutation (Table 4). The majority of the up-regulated transcriptional units is involved in (branched-chain) amino acid metabolism or peptide transport. Some of these (e.g. *dpp, ilv* and *his*) belong to the regulon of the transcriptional regulator CodY, although the extent of derepression seemed less than observed when CodY itself was completely absent (Den Hengst et al, 2005). Interestingly, expression of *cysD, cysK, cysM* and *metB2* was significantly increased in MG*bcaP*. Expression of these genes, encoding enzymes required for methionine and cysteine biosynthesis, has recently been shown to be controlled by the transcriptional activator CmbR (Sperandio et al, 2005). Thus, BcaP provides a link between sulfur amino acid biosynthesis and metabolism of BCAAs in *L. lactis.* **BrnQ contributes to BCAA transport in *L. lactis.*** Although BcaP is responsible for most of the BCAA uptake in *L. lactis* (Fig. 5), MG*bcaP* can still grow in CDM containing only free amino acids (Fig. 2). Since *L. lactis* MG1363 is auxotrophic for BCAAs, the strain must contain at least one other transport system for BCAAs that is active under those conditions. *L. lactis* MG1363 contains a homolog of BrnQ, a low-affinity BCAA carrier in a number of Gram-positive bacteria, among which *Lactobacillus delbruckii* and *Salmonella typhimurium* (Inaoka et al, 2003; Stucky et al, 1995). To investigate whether BrnQ might serve a similar purpose in *L. lactis* MG1363, the gene was deleted from the chromosome and the growth characteristics of the resulting strain (MG*brnQ*) were examined (Fig. 7). Unlike for MG*bcaP,* no significant growth difference was observed between *L. lactis* MG1363 and MG*brnQ* grown in CDM⁷⁵. In contrast, a strain in which both *brnQ* and *bcaP* were deleted (MG*bcaPbrnQ*) was not viable in this medium. After a prolonged lag-phase, the double mutant strain did grow in standard CDM, but growth was significantly hampered (9-fold) and the cultures reached a lower final cell density than the wild type strain (1.5-fold). Growth of the double mutant was enhanced by addition of BCAAs to the CDM. No differences in growth behavior between wild type, mutant and double mutant strains were observed in ½M17 or in CDM supplemented with casitone (data not shown). These results indicate that *brnQ* serves as a second, but less efficient, BCAA transport system in *L. lactis* MG1363.

**TABLE 1. Bacterial strains and plasmids used in this study.**

| **Strain or plasmid** | **Relevant phenotype or genotype** | **Source or reference** |
|---|---|---|
| **Strains** | | |
| MG 1363 | Lac⁻; Prt⁻ ; Plasmid-free derivative of NCDO712 | Gasson, 1983 |
| NZ9000 | MG1363 *pepN::nisRK* | Kuipers et al, 1998 |
| NZ9700 | Nisin-producing transconjugant of MG1363 containing Tn5276 | Kuipers et al, 1993 |
| MGbrnQ | MG1363 derivate, chromosomal deletion of *brnQ* | This work |
| MG*bcaPbrnQ* | MG1363 derivate, chromosomal deletion of *bcaP* and *brnQ* | This work |
| *MGbcaP* | MG1363 derivate, chromosomal deletion of *bcaP* | This work |

| **Plasmids** | | |
|---|---|---|
| pNG8048 | Cm^{r}, Ery^{r}, expression vector carrying the nisin-inducible P*_{nisA}* | Lab collection |
| pNZ9530 | Ery^{r}, *nisRK* cloned in pIL252; constitutive expression of *nisRK* | Kleerebezem et al, 1997 |
| pVE6007 | Cm^{r}; Temp^{s} replication derivate of pWV01 | Maguin et al, 1992 |
| pORI280 | Ery^{r}, *ori*⁺, RepA⁻ ; *lacZ* expressed constitutively from P32 promoter | Leenhouts et al, 1996 |
| pNG*bcaP* | pNG8048 containing *bcaP* gene | This work |
| *pNGbcaP-H6* | pNG8048 containing *bcaP-his6* of *L. lactis* MG1363 behind P*_{nisA}* | This work |
| pORI::*bcaP* | Ery^{r}, LacZ⁺; pORI280 containing *bcaP* deletion construct | This work |
| pORI::*brnQ* | Ery^{r}, LacZ⁺; pORI280 containing *brnQ* deletion construct | This work |
| | Ery^{r}, LacZ⁺; pORI280 containing *bcaP* and *brnQ* deletion | |
| pORI::*bcaPbrnQ* | construct | This work |

**TABLE 2. Oligonucleotides used in this study**

| **Name** | **Sequence (5'-3')^{a}** |
|---|---|
| ctrA-P1 | GCTCTAGACTAGAATTAGGACATATATCAC |
| ctrA-P2 | CGCGGATCCCTCATAAATCCCATAATAAATCCTC |
| ctrA-P3 | CGCGGATCCTGGTTCCTTATTGGAATTGCG |
| ctrA-P4 | CCGGAATTCAACGGCTGGTACGATACGAAC |
| brnQ-P1 | GCTCTAGACATTAGTCCAAATGGCGATACC |
| brnQ-P2 | CGCGGATCCGATAGTCTTTACCAGCTAGTTTC |
| brnQ-P3 | CGCGGATCCGAGCTTCTAATATTTAGGAGCTC |
| brnQ-P4 | CCGGAATTCCATCTCGGTTTTAACGTCTGAAC |
| ctrA-N | CTAGACCACCATGGGATTTATGAGAAAAGCC |
| ctrA-C | CTAGTCTAGACGTCTTATTTCTTTTTGCGACG |
| ctrA-CH6 | CTAGTCTAGATTAGTGATGGTGATGGTGATGTTTCTTTTTGCGACGATTTCCATAA |

| | |
|---|---|
| ^{a}) Restriction enzyme sites are underlined. | |

**TABLE 3. Homologues of L. lactis MG1363 BcaP in several species of Gram-positive bacteria.**

| **Species** | **Protein^{a}** | **Protein length (amino acids)** | **Identity (%)** | **Similarity (%)** | **Reference** |
|---|---|---|---|---|---|
| *Lactococcus lactis* MG1363 | BcaP (CtrA) | 465 | 100 | 100 | this work |
| *Lactococcus lactis* IL1403 | CtrA | 469 | 98 | 99 | Bolotin et al, 2001 |
| *Staphylococcus aureus* COL | YP_187408 | 482 | 64 | 80 | Gill et al, 2005 |
| Leuconostoc mesenteroides subsp. mesenteroides ATCC 8293 | Lmes020010 89 | 466 | 43 | 63 | NCBI Microbial Genomes Annotation Project |
| *Lactobacillus casei* ATCC 334 | ZP_0038437 9 | 464 | 40 | 61 | ^{b}) |
| *Lactobacillus plantarum* WCFS1 | Lp_0861 | 465 | 39 | 61 | Kleerebezem et al, 2003 |
| *Listeria innocua* | Lin0648 | 463 | 36 | 61 | Glaser et al, 2001 |
| *Enterococcus faecalis* V583 | NP_814667 | 463 | 34 | 58 | Paulsen et al, 2003 |
| *Bacillus cereus* G9241 | ZP_0023819 2 | 471 | 37 | 57 | Hoffmaster et al, 2004 |
| *Bacillus subtilis* 168 | YhdG | 465 | 33 | 56 | Kunst et al, 1997 |
| Bifidobacterium longum NCC2705 | BL1150 | 478 | 33 | 54 | JOURNAL Proc. Natl. Acad. Sci. U.S.A. 99 (22), 14422-14427 (2002) |
| *Streptococcus pneumoniae* R6 | YfnA | 467 | 34 | 54 | Hoskins et al, 2001 |
| *Streptococcus thermophilus* CNRZ1066 | str1361 | 461 | 33 | 53 | Bolotin et al, 2004 |

| | | | | | |
|---|---|---|---|---|---|
| ^{a}) In case no protein name was assigned, the locus tag of the encoding ORF is shown. ^{b}) Part of an ongoing genome-sequencing project the sequence data of which is available online (www.doe.jgi.gov). | | | | | |

**TABLE 4. Summary of comparison of the transcriptomes of L. lactis MGbcaP and L. lactis MG1363**

| **Transcriptional unit^{a}** | **Expression Ratio^{b}** | **Significance (*p*-value)** | **Description^{c}** | **Regulon** |
|---|---|---|---|---|
| *dppA, P, B, C*, *D, F* | 4.5 | 10⁻⁸ | dipeptide transport system | CodY |
| ybbE | 3.0 | 10⁻¹³ | hypothetical protein, downstream of *bcaP* | - |
| *llmg_0328* | 2.4 | 10⁻⁶ | hypothetical protein | - |
| *comX* | 2.2 | 10⁻⁹ | putative regulator | - |
| *gltA, citB, icD* | 2.1 | 10⁻¹³ | krebs TCA cycle enzymes | CodY |
| leuC, A | 2.0 | 10⁻⁹ | leucine biosynthesis | CodY |
| *hisC, Z, G, D, B,* | 1.9 | | | |
| *ymdC, hisH, A, F, I,* | | 10⁻¹⁰ | | |
| K | | | histidine biosynthesis | CodY |
| *metC-cysK* | 1.9 | 10⁻⁸ | methionine/cysteine synthesis | CmbR |
| *rmaG* | 1.9 | 10⁻⁴ | transcriptional regulator | - |
| *llmg_1066* | 1.8 | 10⁻⁶ | putative membrane protein | - |
| *llmg_0472*/*0473* | 1.7 | 10⁻⁵ | hypothetical proteins | - |
| *ilvD, B, N, C, A, aldB* | 1.6 | 10⁻⁷ | BCAA biosynthesis | CodY |
| *fabl* | 1.6 | 10⁻⁵ | enoyl-ACP reductase | - |
| *purC,H* | 1.6 | 10⁻⁷ | purine synthesis | CmbR |
| *cysD* | 1.6 | 10⁻¹¹ | O-acetylserine sulthydrylase | CmbR |
| *ydbE* | 1.6 | 10⁻⁴ | hypothetical protein | - |
| *glgD* | 1.6 | 10⁻⁶ | glucose-1-phosphate denylyltransferase | CmbR? |
| *yrbB* | 1.5 | 10⁻¹⁰ | hypothetical protein | - |
| *yriD* | 1.5 | 10⁻⁶ | hypothetical protein | CmbR |
| *cysM* | 1.5 | 10⁻⁷ | cysteine synthase | CmbR |
| *cpo* | -1.5 | 10⁻⁶ | hydrolase or acyltransferase | - |
| *yrcA* | -1.5 | 10⁻⁷ | phospho-beta-glucosidase | - |
| *yjaE* | -1.5 | 10⁻⁴ | hypothetical protein | - |
| *ynjC* | -1.5 | 10⁻⁴ | hypothetical protein | - |
| *argF* | -1.5 | 10⁻⁶ | ornithine carbamoyltransferase | - |
| *yedF* | -1.6 | 10⁻⁷ | PTS system IIABC component | - |
| *trpB* | -1.6 | 10⁻⁶ | tryptophan synthase beta chain | - |
| *butB* | -1.6 | 10⁻⁶ | dehydrogenase | - |
| *ptcA* | -1.6 | 10⁻⁵ | cellobiose-PTS system component | - |
| *panE* | -1.7 | 10⁻⁹ | ketopantoate reductase | - |
| *fruA* | -1.8 | 10⁻⁵ | fructose-PTS system component | - |
| *pgmB* | -1.9 | 10⁻⁹ | beta-phosphoglucomutase | - |
| *busAB* | -1.9 | 10⁻⁸ | betaine ABC transporter | - |
| *cstA* | -1.9 | 10⁻⁴ | carbon starvation protein | - |
| *bglS, ybhD* | -1.9 | 10⁻⁶ | beta-glucosidase A | - |
| *yecA* | -2.1 | 10⁻⁵ | transcriptional regulator | - |
| *chiA, yucG* | -2.3 | 10⁻⁵ | chitinase | - |
| *rcfB, yxbD* | -2.6 | 10⁻⁴ | transcriptional regulator, transporter | - |
| *argH* | -2.8 | 10⁻⁷ | argininosuccinate lyase | - |
| *yjjA* | -3.7 | 10⁻¹⁴ | hypothetical protein | - |

| | | | | |
|---|---|---|---|---|
| ^{a}) The gene (in an operon) that shows the highest ratio of expression is underlined. ^{b}) Ratio of expression in *L. lactis* MG*bcaP* over that in *L. lactis* MG1363. ^{c}) (Putative) gene function. | | | | |

**TABLE 5.**

| **Transcriptional unit^{a}** | **Expression Ratio^{b}** | **Significance (*p*-value)** | **Description^{c}** | **Regulon** |
|---|---|---|---|---|
| ybbE | 3.0 | 10⁻¹³ | hypothetical protein, downstream of *bcaP* | - |
| *llmg_0328* | 2.4 | 10⁻⁶ | hypothetical protein | - |
| *comX* | 2.2 | 10⁻⁹ | putative regulator | - |
| *metC-cysK* | 1.9 | 10⁻⁸ | methionine/cysteine synthesis | CmbR |
| *rmaG* | 1.9 | 10⁻⁴ | transcriptional regulator | - |
| *llmg_1066* | 1.8 | 10⁻⁶ | putative membrane protein | - |
| *llmg_0472*/*0473* | 1.7 | 10⁻⁵ | hypothetical proteins | - |
| *ilvD, B, N, C, A, aldB* | 1.6 | 10⁻⁷ | BCAA biosynthesis | CodY |
| *fabI* | 1.6 | 10⁻⁵ | enoyl-ACP reductase | - |
| *purC, H* | 1.6 | 10⁻⁷ | purine synthesis | CmbR |
| *cysD* | 1.6 | 10⁻¹¹ | O-acetylserine sulfhydrylase | CmbR |
| *ydbE* | 1.6 | 10⁻⁴ | hypothetical protein | - |
| *glgD* | 1.6 | 10⁻⁶ | glucose-1-phosphate denylyltransferase | CmbR? |
| *yrbB* | 1.5 | 10⁻¹⁰ | hypothetical protein | - |
| *yriD* | 1.5 | 10⁻⁶ | hypothetical protein | CmbR |
| *cysM* | 1.5 | 10⁻⁷ | cysteine synthase | CmbR |
| *cpo* | -1.5 | 10⁻⁶ | hydrolase or acyltransferase | - |
| *yrcA* | -1.5 | 10⁻⁷ | phospho-beta-glucosidase | - |
| *yjaE* | -1.5 | 10⁻⁴ | hypothetical protein | - |
| *ynjC* | -1.5 | 10⁻⁴ | hypothetical protein | - |
| *argF* | -1.5 | 10⁻⁶ | ornithine carbamoyltransferase | - |
| *yedF* | -1.6 | 10⁻⁷ | PTS system IIABC component | - |
| *trpB* | -1.6 | 10⁻⁶ | tryptophan synthase beta chain | - |
| *butB* | -1.6 | 10⁻⁶ | dehydrogenase | - |
| *ptcA* | -1.6 | 10⁻⁵ | cellobiose-PTS system component | - |
| *panE* | -1.7 | 10⁻⁹ | ketopantoate reductase | - |
| *fruA* | -1.8 | 10⁻⁵ | fructose-PTS system component | - |
| *pgmB* | -1.9 | 10⁻⁹ | beta-phosphoglucomutase | - |
| *busAB* | -1.9 | 10⁻⁸ | betaine ABC transporter | - |
| *cstA* | -1.9 | 10⁻⁴ | carbon starvation protein | - |
| *bglS, ybhD* | -1.9 | 10⁻⁶ | beta-glucosidase A | - |
| *yecA* | -2.1 | 10⁻⁵ | transcriptional regulator | - |
| *chiA, yucG* | -2.3 | 10⁻⁵ | chitinase | - |
| *rcfB, yxbD* | -2.6 | 10⁻⁴ | transcriptional regulator, transporter | - |
| *argH* | -2.8 | 10⁻⁷ | argininosuccinate lyase | - |
| *yjjA* | -3.7 | 10⁻¹⁴ | hypothetical protein | - |

| | | | | |
|---|---|---|---|---|
| ^{a}) The gene (in an operon) that shows the highest ratio of expression is underlined. ^{b}) Ratio of expression in *L. lactis* MG*bcaP* over that in *L. lactis* MG1363. ^{c}) (Putative) gene function. | | | | |

### Brief description of the drawings

FIG. 1. Nucleotide sequence of the *bcaP* locus and deduced amino acid sequence of the gene product. Putative promoter sequences are underlined and indicated in bold. The CodY-box derivates are overlined and indicated in bold. The RBS is underlined and indicated in italics. The stop codon is indicated by an asterisk. Putative membrane spanning domains are boxed. A putative transcriptional terminator is doubly underlined.
FIG. 2. Growth curves *of L. lactis* MG1363 and its *bcaP* deletion mutant. Growth of *L. lactis* MG1363 and MG*bcaP* in CDM supplemented with casitone (closed and open circles, respectively). *L. lactis* MG1363, MG*bcaP* and MG*bcaP* overproducing BcaP were grown in CDM containing free amino acids as the sole source of amino acids (closed triangles, open triangles and closed squares, respectively). Alternatively, *L. lactis* MG*bcaP* was grown in CDM⁷⁵, a medium in which the amino acid concentration was lowered to 75% of that in CDM (open squares). Cells were grown at 30°C and the absorbance of the culture at 595 nm was measured every 30 min.
FIG. 3. Western detection of BcaP-H6 synthesis. Cells containing pNG*bcaP-H6* were grown in ½GM17 until the exponential phase, after which they were incubated for 2 hrs in the absence (-) or presence (+) of nisin to induce BcaP-H6 synthesis. Samples were collected and used for SDS-PAGE, followed by Western hybridization with HIS-specific antibody as described in Materials and Methods of Example 1.
FIG. 4. Growth of *L. lactis* MG1363 and MG*bcaP* in the presence of different amino acids and dipeptides. (A) Growth *of L. lactis* MG1363 and MG*bcaP* in CDM supplemented with amino acids P/A/H (closed and open circles, respectively) or I/L/V (closed and open triangles, respectively). (B) Growth of *L*. *lactis* MG1363 and MG*bcaP* in CDM supplemented with dipeptides PG/AH (closed and open circles, respectively) or LI/PV (closed and open triangles, respectively). Cells were grown at 30°C and the optical density of the culture at 595 nm was measured every 30 min.
FIG. 5. Time course of uptake of BCAAs and methionine in whole cells of *L. lactis.* Uptake of L-isoleucine by *L. lactis* MG1363 (open circles) and MG*bcaP* (closed circles). Uptake by *L. lactis* MG*bcaP* (pNG*bcaP*), overproducing BcaP, of L-isoleucine (closed triangles), L-leucine (closed squares), L-valine (open triangles) and L-methionine (open squares). Transport assays using concentrated samples of cells harvested from the exponential phase of growth were performed in the presence of 1 µM ¹⁴C- or ³⁵S-labeled amino acid and 250 µM of the corresponding unlabeled amino acid as described in Materials and Methods. Prior to the uptake assay, cells were energized by incubation for 5 min at 30°C in the presence of 0.5% glucose.
FIG. 6. Substrate specificity of *L. lactis* MG1363 BcaP as determined by the inhibition of L-[¹⁴C]isoleucine uptake by L-amino acids. Shown is the percentage of L-[¹⁴C]isoleucine uptake by lactococcal cells overproducing BcaP in the presence of 250 µM unlabeled L-isoleucine (control) and a 10-fold excess of each of the 20 L-amino acids. The uptake was determined after 1 min of incubation at 30°C and the assay was performed as described in the legend to Fig. 5. The means of three independent measurements are shown.
FIG. 7. Growth of *L. lactis* MG1363, MG*brnQ* and MG*bcaPbrnQ* in different media. Growth of *L. lactis* MG1363 (closed circles), MG*brnQ* (open circles) and MG*bcaPbrnQ* (open triangles) in CDM⁷⁵, MG*bcaPbrnQ* (closed triangles) in CDM⁷⁵ supplemented with amino acids I/L/V and MG*bcaPbrnQ* (squares) in standard CDM. Cells were grown at 30°C and the optical density of the culture at 595 nm was measured every 30 min.

### References

Altschul, S. F., W. Gish, W. Miller, E. W. Myers, and D. J. Lipman. 1990. Basic local alignment search tool. J. Mol. Biol. 215:403-410
Bolotin, A., P. Wincker, S. Mauger, O. Jaillon, K. Malarme, J. Weissenbach, S. D. Ehrlich, and A. Sorokin. 2001. The complete genome sequence of the lactic acid bacterium Lactococcus lactis ssp. lactis IL1403. Genome Res. 11:731-753
Bolotin, A., B. Quinquis, P. Renault, A. Sorokin, S. D. Ehrlich, S. Kulakauskas, A. Lapidus, E. Goltsman, M. Mazur, G. D. Pusch, M. Fonstein, R. Overbeek, N. Kyprides, B. Purnelle, D. Prozzi, K. Ngui, D. Masuy, F. Hancy, S. Burteau, M. Boutry, J. Delcour, A. Goffeau, and P. Hols. 2004. Complete sequence and comparative genome analysis of the dairy bacterium Streptococcus thermophilus. Nat. Biotechnol. 22:1554-1558
Den Hengst, C. D., S. A. F. T. van Hijum, J. M. Geurts, A. Nauta, J. Kok, and O. P. Kuipers. 2005. The Lactococcus lactis CodY regulon: identification of a conserved cis-regulatory element. J. Biol. Chem. 280:34332-34342
De Ruyter, P.G., Kuipers, O.P., De Vos, W.M. 1996. Controlled gene expression systems for Lactococcus lactis with the food-grade inducer nisin. Appl Environ Microbiol. 62(10):3662-7
Gasson, M. J. 1983. Plasmid complements of Streptococcus lactis NCDO 712 and other lactic streptococci after protoplast-induced curing. J. Bacteriol. 154:1-9
Gill, S. R., D. E. Fouts, G. L. Archer, E. F. Mongodin, R. T. Deboy, J. Ravel, I. T. Paulsen, J. F. Kolonay, L. Brinkac, M. Beanan, R. J. Dodson, S. C. Daugherty, R. Madupu, S. V. Angiuoli, A. S. Durkin, D. H. Haft, J. Vamathevan, H. Khouri, T. Utterback, C. Lee, G. Dimitrov, L. Jiang, H. Qin, J. Weidman, K. Tran, K. Kang, I. R. Hance, K. E. Nelson, and C. M. Fraser. 2005. Insights on evolution of virulence and resistance from the complete genome analysis of an early methicillin-resistant Staphylococcus aureus strain and a biofilm-producing methicillin-resistant Staphylococcus epidermidis strain. J. Bacteriol. 187:2426-2438
Glaser, P., L. Frangeul, C. Buchrieser, C. Rusniok, A. Amend, F. Baquero, P. Berche, H. Bloecker, P. Brandt, T. Chakraborty, A. Charbit, F. Chetouani, E. Couve, D. A. de, P. Dehoux, E. Domann, G. Dominguez-Bernal, E. Duchaud, L. Durant, O. Dussurget, K. D. Entian, H. Fsihi, P. F. Garcia-del, P. Garrido, L. Gautier, W. Goebel, N. Gomez-Lopez, T. Hain, J. Hauf, D. Jackson, L. M. Jones, U. Kaerst, J. Kreft, M. Kuhn, F. Kunst, G. Kurapkat, E. Madueno, A. Maitournam, J. M. Vicente, E. Ng, H. Nedjari, G. Nordsiek, S. Novella, P. B. de, J. C. Perez-Diaz, R. Purcell, B. Remmel, M. Rose, T. Schlueter, N. Simoes, A. Tierrez, J. A. Vazquez-Boland, H. Voss, J. Wehland, and P. Cossart. 2001. Comparative genomics of Listeria species. Science 294:849-852
Hoffmaster, A. R., J. Ravel, D. A. Rasko, G. D. Chapman, M. D. Chute, C. K. Marston, B. K. De, C. T. Sacchi, C. Fitzgerald, L. W. Mayer, M. C. Maiden, F. G. Priest, M. Barker, L. Jiang, R. Z. Cer, J. Rilstone, S. N. Peterson, R. S. Weyant, D. R. Galloway, T. D. Read, T. Popovic, and C. M. Fraser. 2004. Identification of anthrax toxin genes in a Bacillus cereus associated with an illness resembling inhalation anthrax. Proc. Natl. Acad. Sci. U. S. A 101:8449-8454
Holo, H. and I. F. Nes. 1995. Transformation of Lactococcus by electroporation. Methods Mol. Biol. 47:195-199
Hoskins, J., W. E. Alborn, Jr., J. Arnold, L. C. Blaszczak, S. Burgett, B. S. DeHoff, S. T. Estrem, L. Fritz, D. J. Fu, W. Fuller, C. Geringer, R. Gilmour, J. S. Glass, H. Khoja, A. R. Kraft, R. E. Lagace, D. J. LeBlanc, L. N. Lee, E. J. Lefkowitz, J. Lu, P. Matsushima, S. M. McAhren, M. McHenney, K. McLeaster, C. W. Mundy, T. I. Nicas, F. H. Norris, M. O'Gara, R. B. Peery, G. T. Robertson, P. Rockey, P. M. Sun, M. E. Winkler, Y. Yang, M. Young-Bellido, G. Zhao, C. A. Zook, R. H. Baltz, S. R. Jaskunas, P. R. Rosteck, Jr., P. L. Skatrud, and J. I. Glass. 2001. Genome of the bacterium Streptococcus pneumoniae strain R6. J. Bacteriol. 183:5709-5717
Inaoka, T., K. Takahashi, M. Ohnishi-Kameyama, M. Yoshida, and K. Ochi. 2003. Guanine nucleotides guanosine 5'-diphosphate 3'-diphosphate and GTP co-operatively regulate the production of an antibiotic bacilysin in Bacillus subtilis. J. Biol. Chem. 278:2169-2176
Kleerebezem, M., M. M. Beerthuyzen, E. E. Vaughan, W. M. de Vos, and O. P. Kuipers. 1997. Controlled gene expression systems for lactic acid bacteria: transferable nisin-inducible expression cassettes for Lactococcus, Leuconostoc, and Lactobacillus spp. Appl. Environ. Microbiol. 63:4581-4584
Kleerebezem, M., J. Boekhorst, K. R. van, D. Molenaar, O. P. Kuipers, R. Leer, R. Tarchini, S. A. Peters, H. M. Sandbrink, M. W. Fiers, W. Stiekema, R. M. Lankhorst, P. A. Bron, S. M. Hoffer, M. N. Groot, R. Kerkhoven, V. M. de, B. Ursing, W. M. de Vos, and R. J. Siezen. 2003. Complete genome sequence of Lactobacillus plantarum WCFS1. Proc. Natl. Acad. Sci. U. S. A 100:1990-1995
Kok, J. and G. Buist. 2003. Genetics of proteolysis in Lactococcus lactis, p. 189-224. In Genetics of lactic acid bacteria. Kluwer academics/Plenum publishers, New York
Kuipers, O. P., M. M. Beerthuyzen, R. J. Siezen, and W. M. de Vos. 1993. Characterization of the nisin gene cluster nisABTCIPR of Lactococcus lactis. Requirement of expression of the nisA and nisI genes for development of immunity. Eur. J. Biochem. 216:281-291
Kuipers, O. P., P. G. G. A. de Ruyter, M. Kleerebezem, and W. M. de Vos. 1997. Controlled overproduction of proteins by lactic acid bacteria. Trends Biotechnol. 15:135-140
Kuipers, O. P., P. G. de Ruyter, M. Kleerebezem, and W. M. de Vos. 1998. Quorum sensing controlled gene expression in lactic acid bacteria. Biotechnol 64:15-21
Kunst, F., N. Ogasawara, I. Moszer, A. M. Albertini, G. Alloni, V. Azevedo, M. G. Bertero, P. Bessieres, A. Bolotin, S. Borchert, R. Borriss, L. Boursier, A. Brans, M. Braun, S. C. Brignell, S. Bron, S. Brouillet, C. V. Bruschi, B. Caldwell, V. Capuano, N. M. Carter, S. K. Choi, J. J. Codani, I. F. Connerton, and A. Danchin. 1997. The complete genome sequence of the gram-positive bacterium Bacillus subtilis. Nature 390:249-256
Kyte, J. and R. F. Doolittle. 1982. A simple method for displaying the hydropathic character of a protein. J. Mol. Biol. 157:105-132
Laemmli, U. K. 1970. Cleavage of structural proteins during the assembly of the head of bacteriophage T4. Nature 227:680-685
Leenhouts, K. J., J. Kok, and G. Venema. 1990. Stability of integrated plasmids in the chromosome of Lactococcus Lactis. Appl. Environ. Microbiol 56:2726-2735
Leenhouts, K., G. Buist, A. Bolhuis, A. ten Berge, J. Kiel, I. Mierau, M. Dabrowska, G. Venema, and J. Kok. 1996. A general system for generating unlabelled gene replacements in bacterial chromosomes. Mol. Gen. Genet. 253:217-224
Maguin, E., P. Duwat, T. Hege, D. Ehrlich, and A. Gruss. 1992. New thermosensitive plasmid for gram-positive bacteria. J. Bacteriol. 174:5633-5638
Marchler-Bauer, A., J. B. Anderson, P. F. Cherukuri, C. Weese-Scott, L. Y. Geer, M. Gwadz, S. He, D. I. Hurwitz, J. D. Jackson, Z. Ke, C. J. Lanczycki, C. A. Liebert, C. Liu, F. Lu, G. H. Marchler, M. Mullokandov, B. A. Shoemaker, V. Simonyan, J. S. Song, P. A. Thiessen, R. A. Yamashita, J. J. Yin, D. Zhang, and S. H. Bryant. 2005. CDD: a Conserved Domain Database for protein classification. Nucleic Acids Res. 33:D192-D196
Mierau, I., A. J. Haandrikman, O. Velterop, P. S. Tan, K. L. Leenhouts, W. N. Konings, G. Venema, and J. Kok. 1994. Tripeptidase gene (pepT) of Lactococcus lactis: molecular cloning and nucleotide sequencing of pepT and construction of a chromosomal deletion mutant. J. Bacteriol. 176:2854-2861
Molle, V., Y. Nakaura, R. P. Shivers, H. Yamaguchi, R. Losick, Y. Fujita, and A. L. Sonenshein. 2003. Additional targets of the Bacillus subtilis global regulator CodY identified by chromatin immunoprecipitation and genome-wide transcript analysis. J. Bacteriol. 185:1911-1922
Paulsen, I. T., L. Banerjei, G. S. Myers, K. E. Nelson, R. Seshadri, T. D. Read, D. E. Fouts, J. A. Eisen, S. R. Gill, J. F. Heidelberg, H. Tettelin, R. J. Dodson, L. Umayam, L. Brinkac, M. Beanan, S. Daugherty, R. T. Deboy, S. Durkin, J. Kolonay, R. Madupu, W. Nelson, J. Vamathevan, B. Tran, J. Upton, T. Hansen, J. Shetty, H. Khouri, T. Utterback, D. Radune, K. A. Ketchum, B. A. Dougherty, and C. M. Fraser. 2003. Role of mobile DNA in the evolution of vancomycin-resistant Enterococcus faecalis. Science 299:2071-2074
Sambrook, J., E. F. Fritsch, and T. Maniatis. 1989. Molecular cloning: a laboratory manual. Cold Spring Harbor laboratory Press, Cold Spring Harbor, N.Y.
Slack, F. J., P. Serror, E. Joyce, and A. L. Sonenshein. 1995. A gene required for nutritional repression of the Bacillus subtilis dipeptide permease operon. Mol. Microbiol. 15:689-702
Sonenshein, A. L. 2005. CodY, a global regulator of stationary phase and virulence in Gram-positive bacteria. Curr. Opin. Microbiol. 8:203-207
Sperandio, B., P. Polard, D. S. Ehrlich, P. Renault, and E. Guedon. 2005. Sulfur Amino Acid Metabolism and Its Control in Lactococcus lactis IL1403. J. Bacteriol. 187:3762-3778
Steffes, C., J. Ellis, J. Wu, and B. P. Rosen. 1992. The lysP gene encodes the lysine-specific permease. J. Bacteriol. 174:3242-3249
Stucky, K., A. Hagting, J. R. Klein, H. Matern, B. Henrich, W. N. Konings, and R. Plapp. 1995. Cloning and characterization of brnQ, a gene encoding a low-affinity, branched-chain amino acid carrier in Lactobacillus delbruckii subsp. lactis DSM7290. Mol. Gen. Genet. 249:682-690
Terzaghi, B. E. and W. E. Sandine. 1975. Improved medium for lactic streptococci and their bacteriophages. Appl. Microbiol 29:807-813
Van der Vossen, J.M., Van der Lelie, D., Venema, G. 1987. Isolation and characterization of Streptococcus cremoris Wg2-specific promoters. Appl Environ Microbiol. 53(10):2452-7
van Hijum, S. A., J. A. De, R. J. Baerends, H. A. Karsens, N. E. Kramer, R. Larsen, C. D. den Hengst, C. J. Albers, J. Kok, and O. P. Kuipers. 2005. A generally applicable validation scheme for the assessment of factors involved in reproducibility and quality of DNA-microarray data. BMC. Genomics 6:77

## Claims

1. A method for regulating the amount of at least one branched-chain amino acid in a cell, comprising regulating the amount and/or activity in said cell of a CtrA transporter and/or a homologue thereof, the amount and/or activity in said cell of a *Lactococcus lactis* BrnQ transporter and/or a homologue thereof, and/or the amount and/or activity in said cell of a functional part, derivative and/or analogue of at least one of said transporters and/or homologues.

2. A method according to claim 1, further comprising regulating the amount of at least one sulphur-comprising amino acid in a cell, comprising regulating the amount and/or activity in said cell of a CtrA transporter, a CtrA homologue, and/or a functional part, derivative and/or analogue of said CtrA transporter and/or CtrA homologue.

3. A method according to claim 1 or 2, wherein said CtrA transporter and/or homologue thereof comprises a transporter as depicted in Table 3, preferably a *Lactococcus lactis* CtrA (BcaP) transporter.

4. A method according to any one of claims 1-3, wherein the presence of at least one branched chain amino acid in said cell is counteracted.

5. A method according to any one of claims 1-4, wherein the presence, amount and/or activity in said cell of said transporter and/or homologue is counteracted.

6. A method according to any one of claims 1-5, wherein the amount of said transporter and/or homologue thereof is decreased by functionally deleting at least part of a gene encoding said transporter and/or homologue.

7. A method according to any one of claims 1-6, wherein the amount of said transporter and/or homologue thereof is decreased by downregulating expression of a nucleic acid sequence encoding said transporter and/or homologue.

8. A method according to any one of claims 1-7, wherein the activity of said transporter and/or homologue thereof is decreased by providing said cell with a compound capable of at least partially inhibiting a transportation activity of said transporter and/or homolog.

9. A method according to any one of claims 1-4, wherein the presence, amount and/or activity in said cell of said transporter and/or homologue thereof is induced and/or enhanced.

10. A method according to claim 9, wherein the amount of said transporter and/or homologue, or the amount of a functional part, derivative and/or analogue thereof, is increased by providing said cell with a nucleic acid sequence encoding said transporter and/or homologue, or encoding a functional part, derivative and/or analogue thereof.

11. A method according to claim 9 or 10, wherein the amount of said transporter and/or homologue, or a functional part, derivative and/or analogue thereof, is increased by at least in part inhibiting binding of CodY to a CodY binding domain capable of regulating expression of said transporter, functional part, derivative and/or analogue thereof.

12. A method for regulating branched chain amino acid metabolism and/or sulphur amino acid metabolism and/or dipeptide transport of a cell comprising regulating the amount of at least one branched-chain amino acid and/or sulphur-comprising amino acids in said cell with a method according to any one of claims 1-11.

13. A method for enhancing flavour formation by a cell comprising diminishing the amount of at least one branched chain amino acid in said cell as compared to a situation wherein flavour formation is not enhanced.

14. A method according to any one of claims 1-13, wherein the concentration of branched amino acids is kept and/or brought below 8 mM, more preferably below 4 mM, in *Lactococcus lactis* and/or below 2 mM, more preferably below below 1.5 mM, in Bacillus subtilis.

15. A method according to any one of claims 1-14, wherein said cell comprises a micro-organism, preferably a micro-organism capable of fermenting milk or a milk-derived product, most preferably a Lactococcus, Leuconostoc, Streptococcus, Lactobacillus, Propionibacterium, Bifidobacterium, Pediococcus and/or Carnobacterium.

16. A method according to any one of claims 9-15, comprising:
- enhancing the amount and/or activity of a CtrA transporter and/or a homologue thereof, and/or the amount and/or activity of a BrnQ transporter and/or a homologue thereof, in a micro-organism capable of fermenting milk or a milk-derived product; and
- inoculating milk or a milk-derived product with said micro-organism.

17. A method for producing a dairy product comprising:
- inoculating a starter medium with a culture capable of converting a starter medium into a dairy product, wherein the amount and/or activity of a CtrA transporter and/or a homologue thereof, and/or the amount and/or activity of a BrnQ transporter and/or a homologue thereof, in said culture is increased, and
- fermenting said starter medium.

18. A method for regulating expression of a CodY controlled nucleic acid sequence in a cell, comprising regulating the amount and/or activity in said cell of a CtrA transporter and/or a homologue thereof, the amount and/or activity in said cell of a BrnQ transporter and/or a homologue thereof, and/or the amount and/or activity in said cell of a functional part, derivative and/or analogue of at least one of said transporters and/or homologues.

19. A method for regulating expression in a cell of at least one gene depicted in Table 4, comprising regulating the amount and/or activity in said cell of a CtrA transporter and/or a homologue thereof, the amount and/or activity in said cell of a BrnQ transporter and/or a homologue thereof, and/or the amount and/or activity in said cell of a functional part, derivative and/or analogue of at least one of said transporters and/or homologues.

20. A method for regulating expression in a cell of at least one gene depicted in Table 5, comprising regulating the amount of at least one branched-chain amino acid and/or sulphur-comprising amino acid in said cell.

21. An isolated or recombinant cell comprising a diminished amount and/or activity of a CtrA transporter and/or a homologue thereof, and/or an altered amount and/or activity of a BrnQ transporter and/or a homologue thereof, as compared to a natural situation.

22. An isolated or recombinant cell according to claim 21, wherein at least part of a nucleic acid sequence encoding a CtrA transporter and/or a homologue thereof, and/or a BrnQ transporter and/or homologue thereof, is functionally deleted.

23. An isolated or recombinant cell according to claim 21 or 22, wherein expression of a CtrA transporter and/or a homologue thereof, and/or expression of a BrnQ transporter and/or a homologue thereof, is downregulated.

24. An isolated or recombinant cell comprising an exogenous nucleic acid sequence encoding a CtrA transporter and/or a homologue thereof, and/or encoding a BrnQ transporter and/or a homologue thereof, and/or encoding a functional part, derivative and/or analogue of at least one of said transporters and/or homologues.

25. An isolated or recombinant cell obtainable by a method according to any one of claims 1-16.

26. A dairy product comprising a micro-organism wherein the amount of at least one branched chain amino acid is decreased as compared to a natural micro-organism.

27. A dairy product comprising a micro-organism wherein the amount and/or activity of a CtrA transporter and/or a homologue thereof, and/or the amount and/or activity of a BrnQ transporter and/or a homologue thereof, in said micro-organism is increased as compared to a natural micro-organism.

28. A dairy product according to claim 26 or 27, wherein said micro-organism comprises a culture capable of converting a starter medium into a dairy product, preferably a Lactococcus, Leuconostoc, Streptococcus, Lactobacillus, Propionibacterium, Bifidobacterium, Pediococcus and/or Carnobacterium, more preferably a Lactococcus, most preferably *Lactococcus lactis.*

29. Use of a compound capable of regulating the amount and/or activity of a CtrA transporter and/or a homologue thereof, and/or the amount and/or activity of a BrnQ transporter and/or a homologue thereof, for regulating the amount of at least one branched-chain amino acid and/or sulphur-comprising amino acids in a cell.

30. A bacterial cell, preferably a Lactococcus, Leuconostoc, Streptococcus, Lactobacillus, Propionibacterium, Bifidobacterium, Pediococcus and/or Carnobacterium, more preferably a Lactococcus cell, more preferably a *L.lactis* cell, whose ctrA gene and/or brnQ gene is at least in part functionally deleted.
